# EUROPEAN PATENT APPLICATION

(11) **EP 4 678 737 A1**
(43) Date of publication of application: **14.01.2026**
(21) Application number: 24767087.0
(22) Date of filing: 01.03.2024
(51) Int. Cl.: C12N 5/0735, C12N 5/10

(54) **METHOD FOR PRODUCING PRIMED PLURIPOTENT STEM CELLS**

(30) Priority: 03.03.2023 JP 2023032539
(71) Applicant: Kyoto University, Kyoto-shi, Kyoto 606-8501 (JP)
(72) Inventor: TAKASHIMA, Yasuhiro, Kyoto-shi, Kyoto 606-8501 (JP); IEMURA, Yoshiki, Kyoto-shi, Kyoto 606-8501 (JP); SATO, Masae, Kyoto-shi, Kyoto 606-8501 (JP); TAKAHASHI, Jun, Kyoto-shi, Kyoto 606-8501 (JP); DOI, Daisuke, Kyoto-shi, Kyoto 606-8501 (JP); ETO, Koji, Kyoto-shi, Kyoto 606-8501 (JP); NAKAMURA, Sou, Kyoto-shi, Kyoto 606-8501 (JP); KANEKO, Shin, Kyoto-shi, Kyoto 606-8501 (JP); IKEYA, Makoto, Kyoto-shi, Kyoto 606-8501 (JP); SAITO, Hirohide, Kyoto-shi, Kyoto 606-8501 (JP); FUJITA, Yoshihiko, Kyoto-shi, Kyoto 606-8501 (JP); KASWANDY, Belinda Yunita, Kyoto-shi, Kyoto 606-8501 (JP); WANG, Bo, Kyoto-shi, Kyoto 606-8501 (JP); ZHANG, Chaoqi, Kyoto-shi, Kyoto 606-8501 (JP)
(74) Representative: Hoffmann Eitle
(86) International application number: PCT/JP2024/007912
(87) International publication number: WO 2024/185719

(57) **Abstract**

The present invention provides a method for producing primed pluripotent stem cells, the method comprising: (1) a step of preparing naive pluripotent stem cells of primate animals; and (2) a step of inducing primed pluripotent stem cells from the naive pluripotent stem cells prepared in step (1).

## Description

### FIELD

The present invention relates to a method for producing primed pluripotent stem cells. More specifically, the present invention relates to a method for producing primed pluripotent stem cells comprising a step of inducing primed pluripotent stem cells from primate naive pluripotent stem cells.

### BACKGROUND

During the developmental stage of mammals, an inner cell mass is formed inside the blastocyst, resulting in emergence of an epiblast and a primitive endoderm. The epiblast differentiates into the embryonic mesendoderm through the neuroectoderm and germ cells as well as the primitive gut formation, while the primitive endoderm differentiates into extraembryonic tissue and particularly the yolk sac. The epiblast of the preimplantation embryo comprises cells with the lowest level of DNA methylation, but as development proceeds the level of DNA methylation increases, thus limiting the differentiation potency. The conventional ES cells and iPS cells from primates such as humans are classified as developed prime types, and their differentiation into placenta or primitive endoderm has been difficult to achieve. They are also known to have a high level of DNA methylation.

Previously, Takashima, one of the present inventors, succeeded in obtaining naive pluripotent stem cells (reset cells) by expressing the two genes NANOG and KLF2 in primed human pluripotent stem cells, resetting the human pluripotent stem cells to the same state as the epiblast of a blastocyst (NPL 1). In addition, a different method for inducing naive pluripotent stem cells has been reported, as a method of culturing primed pluripotent stem cells in the presence of 5 types of kinase inhibitors, LIF and activin A (5i/LIF/A) (NPL 2), and a method of culturing in the presence of a transient histone deacetylase inhibitor (NPL 3). The naive pluripotent stem cells produced in this manner can be induced to differentiate into trophectoderm cells, as the starting point for placental cells (PTL 1), but they have been problematic due to their unstable nature and the difficulty and high cost of maintenance culture, as well as being unsuitable for direct application of differentiation-inducing methods used for primed pluripotent stem cells. For these reasons, primed pluripotent stem cells are currently used for almost all purposes involving regular research and regenerative medicine.

### [CITATION LIST]

### [PATENT LITERATURE]

[PTL 1] International Patent Publication No. WO2021/106765

### [NON PATENT LITERATURE]

[NPL 1] Takashima Y. et al., Cell. 158(6):1254-1269 (2014)
[NPL 2] Theunissen T.W. et al., Cell Stem Cell. 15(4):471-487 (2014)
[NPL 3] Guo G. et al., Development. 144(15):2748-2763 (2017)

### SUMMARY

### [TECHNICAL PROBLEM]

As mentioned above, the pluripotent stem cells currently in use are "primed" post-implantation embryo pluripotent stem cells, which are not reprogrammed to be pre-implantation embryos. Therefore, in some cases the epigenome is not completely reset and epigenomic memory remains, which is thought to be a possible reason for different differentiation biases between existing pluripotent stem cell lines. It is an object of the present invention to provide a method for improving the differentiation potency of conventional pluripotent stem cells (in other words, "a method for producing pluripotent stem cells with increased differentiation potency").

### [SOLUTION TO PROBLEM]

During the course of ongoing research on naive pluripotent stem cells, the present inventors discovered that when primed pluripotent stem cells are transiently induced to become naive pluripotent stem cells, and then re-induced back to primed pluripotent stem cells, their differentiation resistance is markedly improved over the primed pluripotent stem cells before naive induction. Moreover, it was found that when somatic cells are directly induced to naive pluripotent stem cells without passing through the cell state equivalent to that of primed cells, and the naive pluripotent stem cells are then induced to primed pluripotent stem cells, the differentiation potency is more excellent than when directly inducing somatic cells to primed pluripotent stem cells. The present invention has been completed as a result of further research conducted on the basis of this finding.

Specifically, the present invention is as follows.
[1] A method for producing a primed pluripotent stem cell, comprising:
   (1) a step of preparing a primate naive pluripotent stem cell, and
   (2) a step of inducing a primed pluripotent stem cell from the naive pluripotent stem cell prepared in step (1).
[2] The method according to [1], comprising a step of collecting the primed pluripotent stem cell obtained in step (2).
[3] The method according to [1] or [2], wherein step (1) is a step of culturing a primate primed pluripotent stem cell to produce a naive pluripotent stem cell.
[4] The method according to [3], wherein the culturing is carried out for 14 days or longer.
[5] The method according to [3] or [4], comprising a step of sorting a CD320-positive cell from the cell population.
[6-1] The method according to [1] or [2], wherein step (1) is a step of producing a naive pluripotent stem cell from a somatic cell without transitioning through a primed pluripotent stem cell.
[6-2] The method according to [1] or [2], wherein the naive pluripotent stem cell prepared in step (1) is produced from a somatic cell without transitioning through a primed pluripotent stem cell.
[6-3] The method according to [1] or [2], wherein the naive pluripotent stem cell prepared in step (1) is produced by introducing a miRNA belonging to the let-7-5p miRNA family into a somatic cell.
[6-4] The method according to [6-3], wherein the miRNA belonging to the let-7-5p miRNA family is miR-98-5p.
[7] The method according to [6-1], comprising a step of introducing a reprogramming factor and a miRNA belonging to the let-7-5p miRNA family into a somatic cell.
[8] The method according to [7], wherein the miRNA belonging to the let-7-5p miRNA family is at least one type of miRNA selected from the group consisting of miR-98-5p, let-7a-5p, let-7b-5p, let-7c-5p, let-7e-5p, let-7f-5p, let-7g-5p and let-7i-5p.
[9-1] The method according to any one of [6-1] to [8], comprising a step of introducing a bone morphogenetic protein into a cell.
[9-2] The method according to [9-1], wherein the bone morphogenetic protein is BMP4.
[10] The method according to any one of [6-1] to [9-2], comprising a step of activating a bone morphogenetic protein in a cell.
[11-1] The method according to any one of [6-1] to [10], comprising a step of introducing a viral vector having a reprogramming factor into a somatic cell.
[11-2] The method according to [11-1], wherein the viral vector is a Sendai virus vector.
[12] The method according to any one of [1] to [11-2], wherein step (2) comprises a step of culturing the naive pluripotent stem cell prepared in step (1) for 4 days or longer in medium containing bFGF.
[13-1] The method according to any one of [1] to [12], wherein step (2) is culturing under a feeder-free condition.
[13-2] The method according to any one of [1] to [13-1], wherein the pluripotent stem cell prepared in step (1) is a pluripotent stem cell established under a feeder-free condition.
[13-3] The method according to any one of [1] to [13-2], wherein step (2) is culturing under a xeno-free condition.
[13-4] The method according to any one of [1] to [13-3], wherein the pluripotent stem cell prepared in step (1) is a pluripotent stem cells established under a xeno-free condition.
[14] The method according to any one of [1] to [13-4], wherein the entire culturing period is under feeder-free and xeno-free conditions.
[15-1] The method according to any one of [1] to [14], wherein the pluripotent stem cell is an induced pluripotent stem cell.
[15-2] The method according to any one of [1] to [15-1] above, wherein the pluripotent stem cell is a cell derived from a human.
[16] A primed pluripotent stem cell obtained by the method according to any one of [1] to [15-2].
[17] A method for producing a cell or an organoid, comprising a step of inducing differentiation of the primed pluripotent stem cell according to [16].
[18] The method according to [17], wherein the cell is selected from the group consisting of a neural crest cell, a neural progenitor cell, a neuron, a hematopoietic progenitor cell, platelet, a T cell, an epiblast-like cell, a primordial germ cell and a myocardial cell.
[19-1] The method according to [17], wherein the organoid is a cerebral cortex organoid.
[19-2] The method according to any one of [17] to [19-1], wherein the step of inducing differentiation is carried out under a feeder-free condition.
[19-3] The method according to any one of [17] to [19-2], wherein the step of inducing differentiation is carried out under a xeno-free condition.
[20] A method for increasing the differentiation potency of a pluripotent stem cell, comprising:
   (1) a step of inducing a naive pluripotent stem cell from a primate primed pluripotent stem cell, and
   (2) a step of inducing a primed pluripotent stem cell from the naive pluripotent stem cell induced in step (1).
[21] The method according to [20], comprising a step of collecting the pluripotent stem cell cultured in step (2).
[22] The method according to [20] or [21], wherein step (1) is carried out for 14 days or longer.
[23] The method according to any one of [20] to [22], wherein step (1) comprises a step of sorting a CD320-positive cell from the cell population.
[24] The method according to any one of [20] to [23], wherein step (2) comprises a step of culturing the naive pluripotent stem cell induced in step (1) for 4 days or longer in medium containing bFGF.
[25-1] The method according to any one of [20] to [23], wherein step (1) and/or step (2) is culturing under a feeder-free condition.
[25-2] The method according to any one of [20] to [25-1], wherein step (1) and/or step (2) is culturing under a xeno-free condition.
[26-1] The method according to any one of [20] to [25-2], wherein the pluripotent stem cell is an induced pluripotent stem cell.
[26-2] The method according to any one of [20] to [26-1], wherein the pluripotent stem cell is a cell derived from a human.
[27] A cell or an organoid produced by the method according to any one of [17] to [19-3].
[28] A medicament containing a cell or an organoid according to [16] or [27].
[29] The medicament according to [28], which is for immunotherapy or cell grafting treatment.

### [ADVANTAGEOUS EFFECTS OF INVENTION]

According to the present invention, it is possible to produce pluripotent stem cells with superior differentiation potency. Furthermore, since it is possible to increase the differentiation potency even of pluripotent stem cells having low differentiation potency, thus reducing the difference in differentiation potency between existing lines of pluripotent stem cells, especially iPS cells, and making the cells easier to induce differentiation into a variety of cell types, the present invention is useful for immunotherapy, regenerative medicine and innovative drug discovery screening.

### BRIEF DESCRIPTION OF DRAWINGS

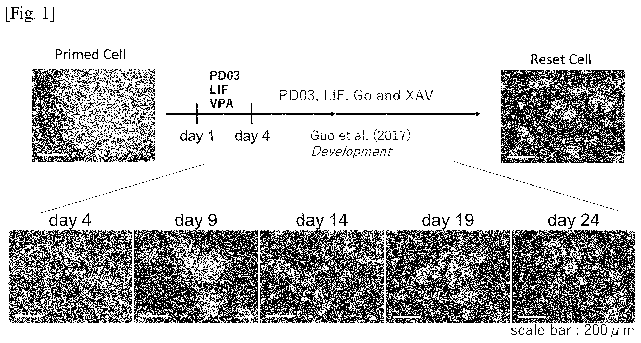

Fig. 1 is a photomicrograph showing changes in cell morphology during the course of induction from primed pluripotent stem cells to naive stem cells.

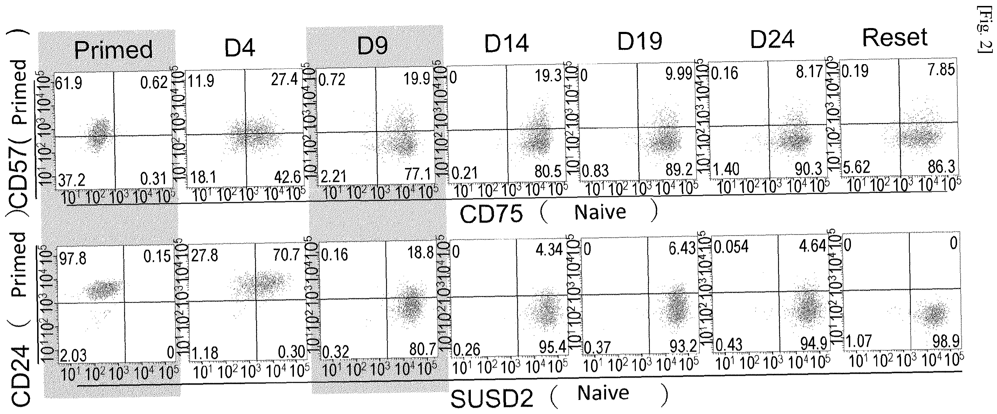

Fig. 2 is a diagram showing the results of flow cytometry analysis of time-dependent change in surface antigens during the course of induction from primed pluripotent stem cells to naive pluripotent stem cells. Most of the cells on day 9 (D9) had lost primed-type markers and expressed naive markers.

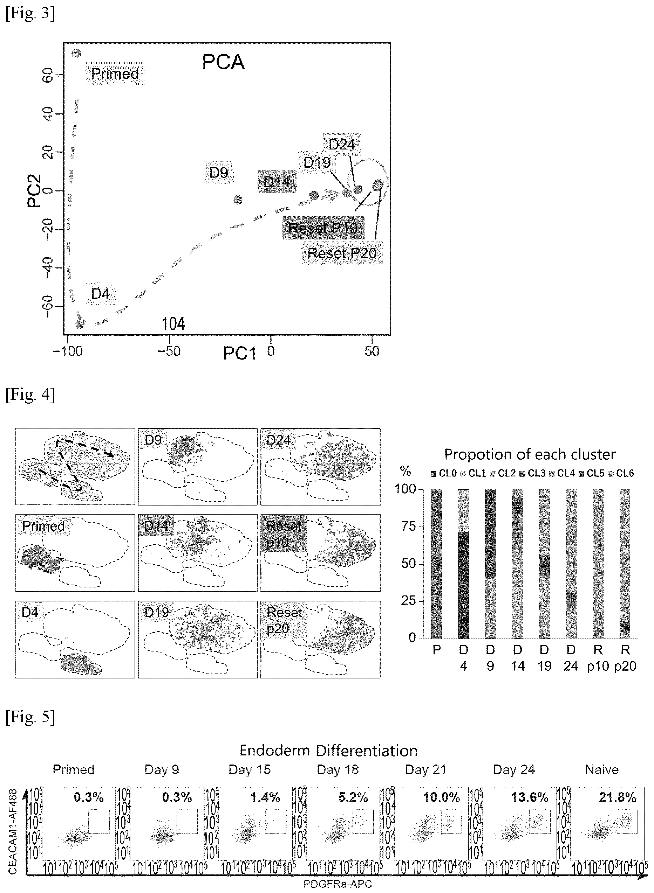

Fig. 3 is a graph showing the results of PCA single cell analysis of time-dependent change in gene expression during the course of induction from primed pluripotent stem cells to naive pluripotent stem cells.
Fig. 4 is a diagram showing the results of single cell analysis of time-dependent change and graphical representation of the proportion of transition of each cluster, during the course of induction from primed pluripotent stem cells to naive pluripotent stem cells.
Fig. 5 shows results of flow cytometry analysis of primitive endoderm differentiation efficiency at different days after induction to naive pluripotent stem cells. According to scRNA-seq, this coincided with the period in which reset cells seemed to appear, indicating that cells are able to differentiate into the primitive endoderm.

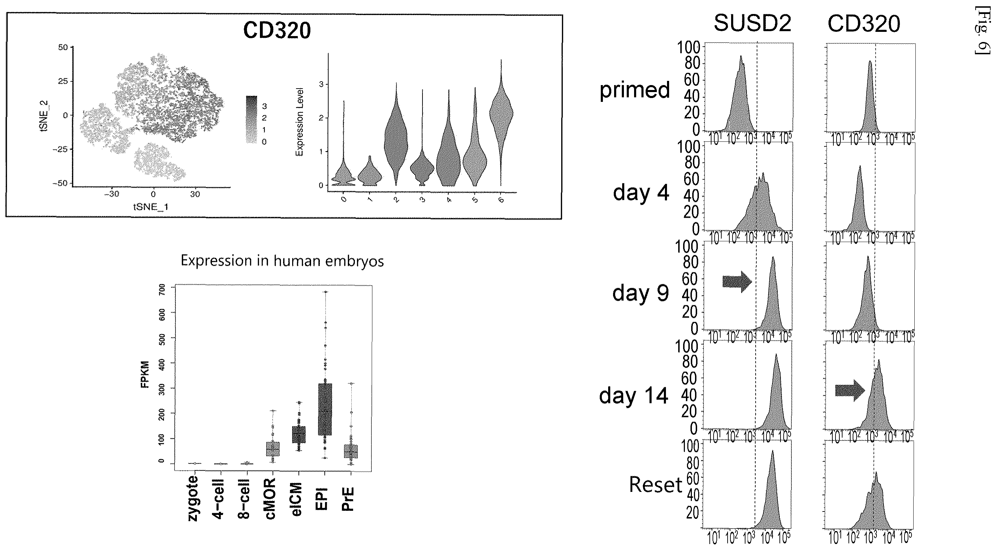

Fig. 6 is a set of diagrams showing the results of single cell analysis and flow cytometry analysis of appearance of the naive marker CD320 during the course of induction to naive pluripotent stem cells.

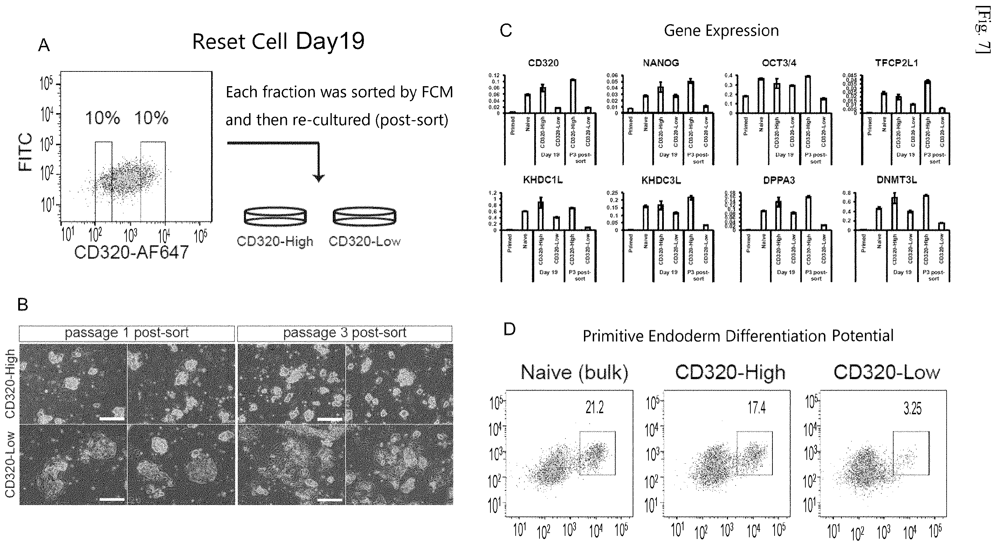

Fig. 7 is a set of diagrams showing cell morphology of CD320-high positive cells among naive pluripotent stem cells under a microscope, results of qPCR expression analysis of naive marker genes, and results of flow cytometry of primitive endoderm differentiation potency. More specifically, the results were obtained by separating CD320-high positive cells (CD320-High) and CD320-low positive cells (CD320-Low) from a cell population on the 19th day (Day 19) after induction to naive pluripotent stem cells, and analyzing expression of pluripotent stem cell marker genes and differentiation potency to primitive endoderm. A: Dot plot of flow cytometry analysis of CD320 expression level in the cells on Day 19. The two blocked regions indicate CD320-High (right) and CD320-Low (left) cell populations. B: Phase contrast microscope photographs after culturing the CD320-High and CD320-Low cells separated in A, showing passage 1 (left panel) and passage 3 (right panel). C: Graphs showing results of qPCR analysis of the expression levels of marker genes for CD320-High and CD320-Low cells. Shown in the gene expression graphs (C) are expression levels for each gene in primed pluripotent stem cells, naive pluripotent stem cells, CD320-high positive cells (Day 19), CD320-low positive cells (Day 19), CD320-high positive cells (passage 3, post-sort) and CD320-low positive cells (passage 3, post-sort), from left. (N=2 for each). Error bars: SE. D: Results of inducing differentiation of unsorted (Bulk), CD320-High cells and CD320-Low cells to primitive endoderm, and flow cytometry analysis based on expression of primitive endoderm markers. The squared off regions in the dot plots represent cells differentiated to primitive endoderm.

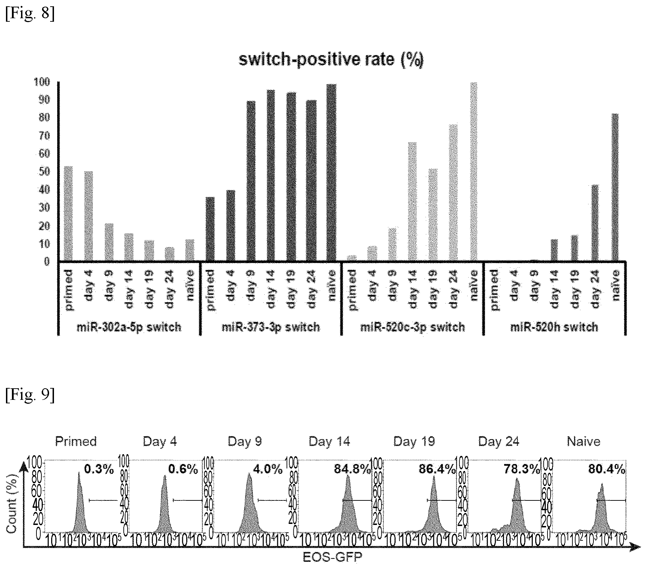

Fig. 8 shows time-dependent change during the course of induction from primed pluripotent stem cells to naive pluripotent stem cells.
Fig. 9 shows results of flow cytometry analysis of changes in expression by EOS-GFP-positive cells during the course of induction from primed pluripotent stem cells to naive pluripotent stem cells.

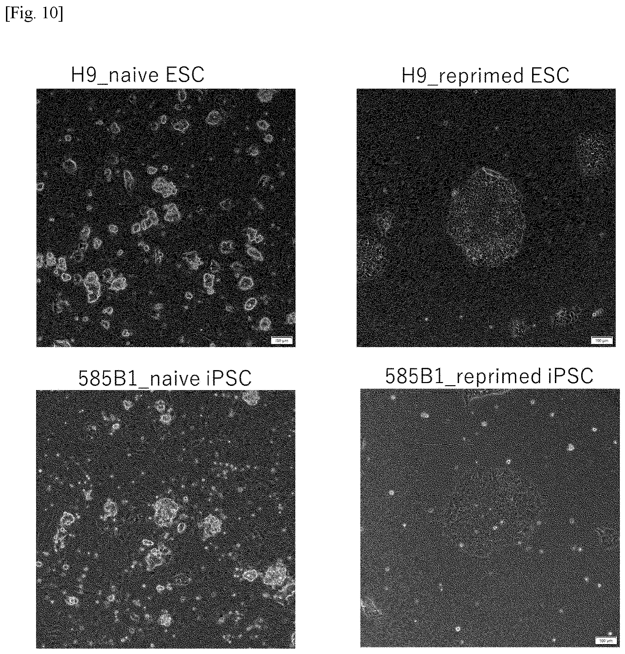

Fig. 10 is a set of microscope photographs showing results observed after induction of primed pluripotent stem cells from naive pluripotent stem cells. Scale bars: 100 µM.

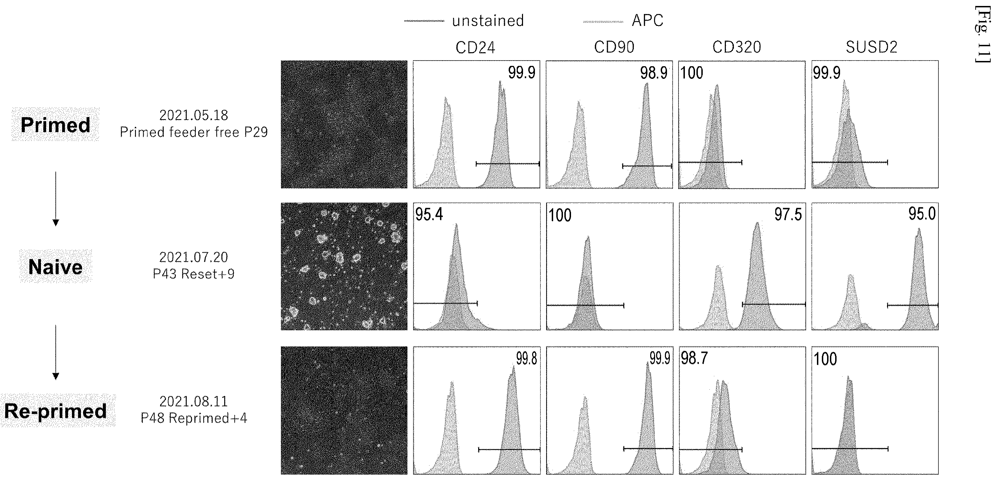

Fig. 11 is set of graphs showing the results of flow cytometry analysis of time-dependent change in surface antigens during the course of induction from primed pluripotent stem cells to naive pluripotent stem cells, and re-induction back to primed pluripotent stem cells.

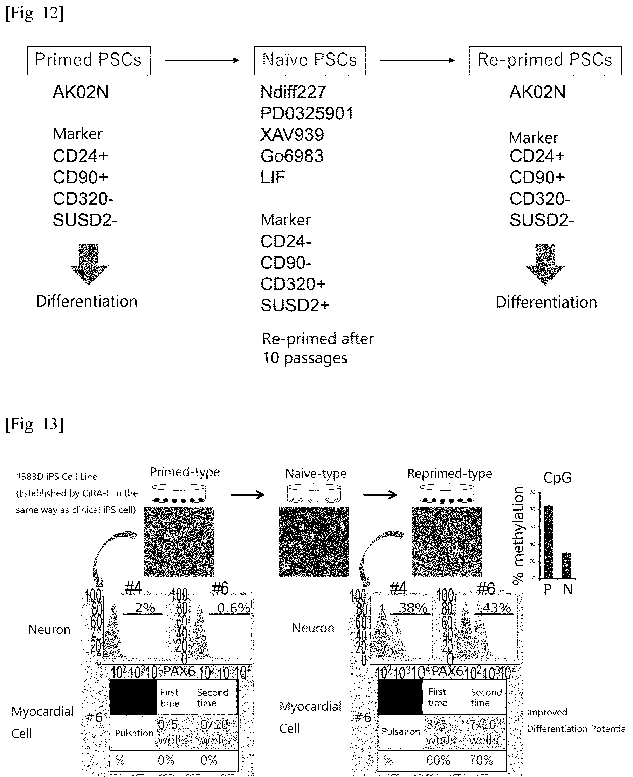

Fig. 12 is a schematic diagram showing the medium, reagents and surface markers used during the course of induction from primed pluripotent stem cells to naive pluripotent stem cells, and re-induction back to primed pluripotent stem cells.
Fig. 13 illustrates induction from primed pluripotent stem cells to naive pluripotent stem cells, and re-induction back to primed pluripotent stem cells. The diagrams also show results of flow cytometry analysis after inducing differentiation of the cells to neurons, and the results of analyzing the ratio of pulsation after inducing differentiation to myocardial cells.

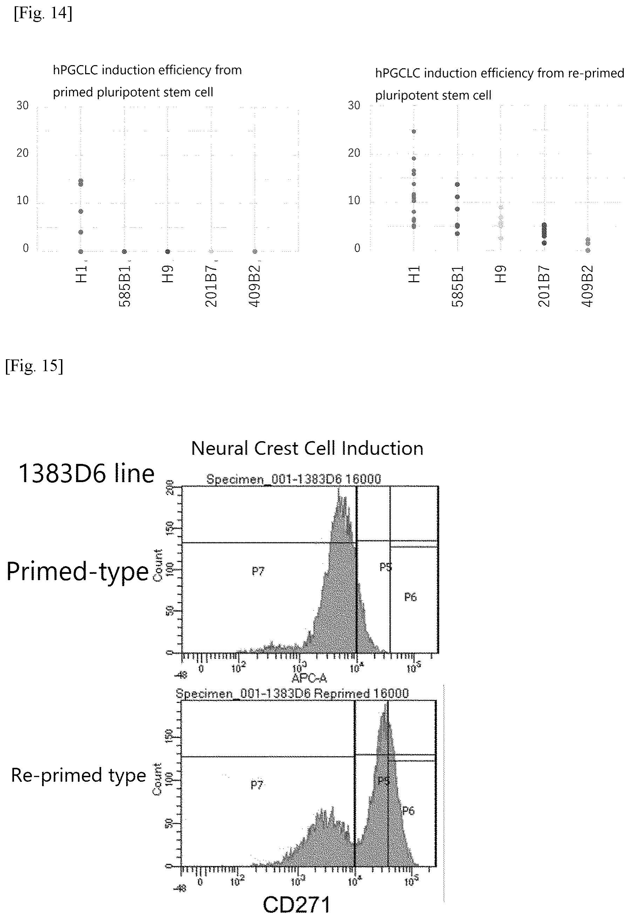

Fig. 14 illustrates induction from primed pluripotent stem cells to naive pluripotent stem cells, and re-induction back to primed pluripotent stem cells. Shown are the results of flow cytometry analysis after inducing differentiation of the cells to primordial germ cells.
Fig. 15 illustrates induction from primed pluripotent stem cells to naive pluripotent stem cells, and re-induction back to primed pluripotent stem cells. Shown are the results of flow cytometry analysis after inducing differentiation of the cells to neural crest cells.

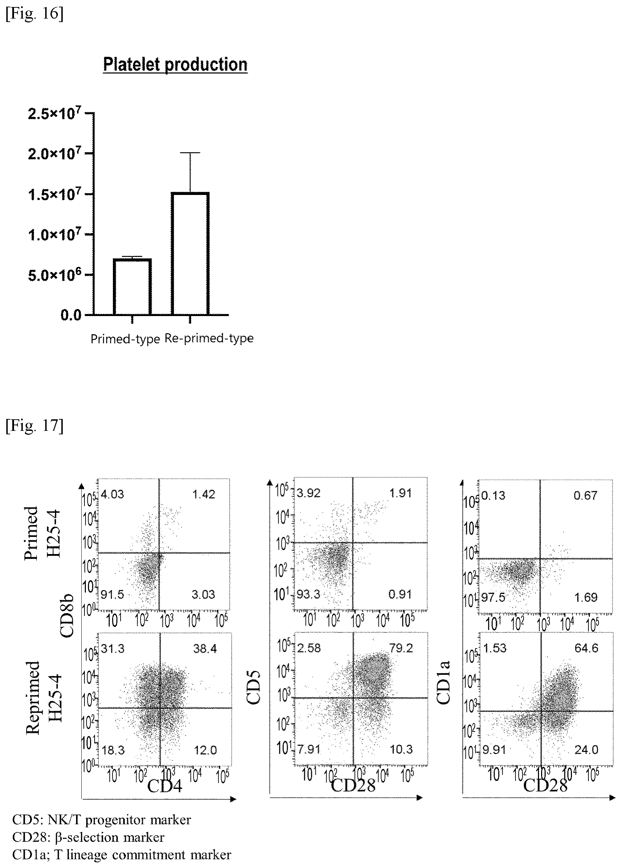

Fig. 16 illustrates induction from primed pluripotent stem cells to naive pluripotent stem cells, and re-induction back to primed pluripotent stem cells. Shown are the results of flow cytometry analysis of the number of platelets produced after induction of the cells into a megakaryocyte line (imMKCL) and subsequent maturation culture.
Fig. 17 illustrates induction from primed pluripotent stem cells to naive pluripotent stem cells, and re-induction back to primed pluripotent stem cells. Shown are the results of flow cytometry analysis of the proportion of induced cells after induction of the cells to T cell precursors (DP cells: CD4-positive, CD8b-positive). The X and Y axis of the plot represent the expression levels of CD4, CD8b, CD5, CD28 and CD1a molecules on the surfaces of T cells. The Original H25-4 at the top represents the T cell precursors from primed pluripotent stem cells, while the Reprimed H25-4 at the bottom represents the T cell precursors induced after re-differentiation to primed pluripotent stem cells by way of induction to naive pluripotent stem cells.

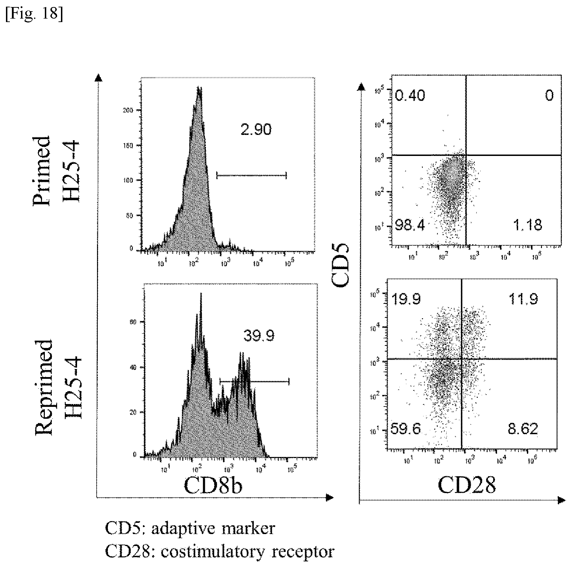

Fig. 18 illustrates induction from primed pluripotent stem cells to naive pluripotent stem cells, and re-induction back to primed pluripotent stem cells. Shown are the results of flow cytometry analysis of the proportion of induced cells after induction of the cells to T cell precursors (DP cells), and induction to mature T cells (CD8SP cells: CD5-positive/CD28-positive, CD8-positive). The X-axis of the histogram and the X and Y axis of the plots represent the expression levels of CD8b, CD5 and CD28 molecules on the surfaces of mature T cells. The Original H25-4 at the top represents the mature T cells from primed pluripotent stem cells, while the Reprimed H25-4 at the bottom represents the mature T cells induced after re-differentiation to primed pluripotent stem cells by way of induction to naive pluripotent stem cells.

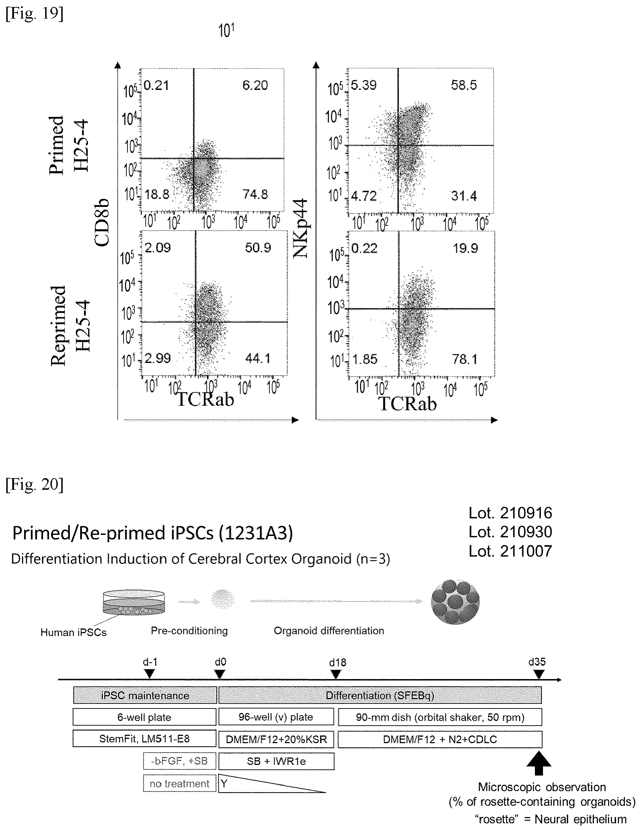

Fig. 19 illustrates induction from primed pluripotent stem cells to naive pluripotent stem cells, and re-induction back to primed pluripotent stem cells. Shown are the results of flow cytometry analysis after induction of the cells to T cells (DP cells), and further induction to mature T cells (CD8SP cells), followed by expansion culturing. The Original H25-4 at the top represents the mature T cells from primed pluripotent stem cells, while the Reprimed H25-4 at the bottom represents the mature T cells induced after re-differentiation to primed pluripotent stem cells by way of induction to naive pluripotent stem cells. The plots represent the expression levels of CD8b and T cell receptor (TCRab) which are expressed on the surfaces of mature T cells, and NKp44 molecule which is not expressed in mature T cells.
Fig. 20 illustrates induction from primed pluripotent stem cells to naive pluripotent stem cells, and re-induction back to primed pluripotent stem cells. Shown is a schematic diagram illustrating the medium, reagents and evaluation method used during the course of inducing differentiation of the cells to a cerebral cortex organoid.

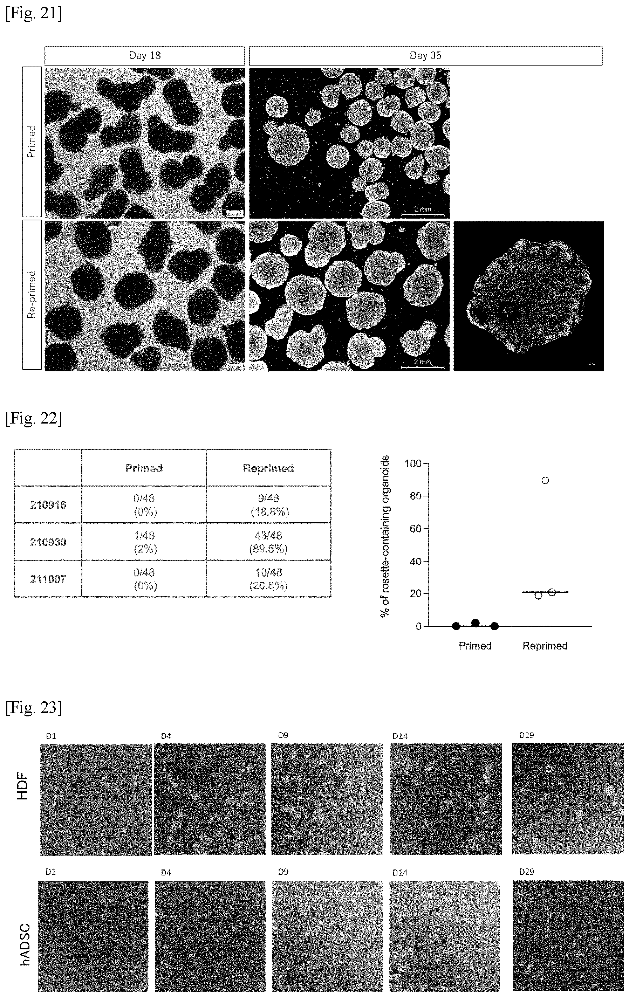

Fig. 21 illustrates induction from primed pluripotent stem cells (top) to naive pluripotent stem cells, and then re-induction back to primed pluripotent stem cells (bottom). Shown are photomicrographs of cell morphology in a bright field and immunostaining with FOXG1/CTIP2/PAX6/DAPI, after inducing differentiation of the cells to a cerebral cortex organoid.
Fig. 22 illustrates induction from primed pluripotent stem cells (left) to naive pluripotent stem cells, and then re-induction back to primed pluripotent stem cells (right). The graph shows the percentage of rosette-containing organoid ("% of rosette-containing organoids") (cerebral cortex organoids) differentiated from the cells.
Fig. 23 is a set of photomicrographs showing changes in cell morphology during the course of induction from somatic cells to naive pluripotent stem cells.

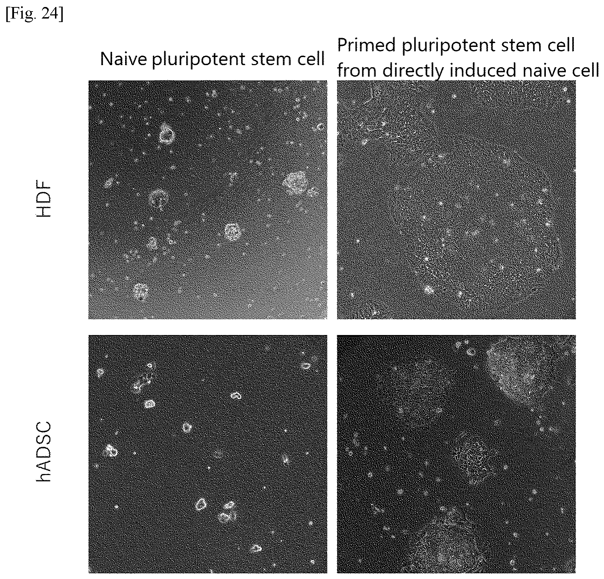

Fig. 24 is a set of photomicrographs showing changes in cell morphology after induction of somatic cells to naive pluripotent stem cells, and re-induction to primed pluripotent stem cells.

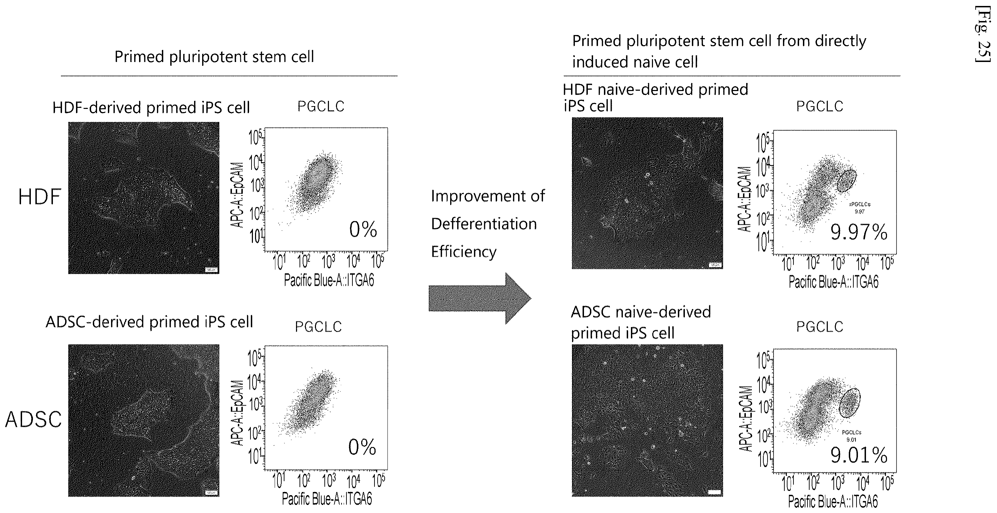

Fig. 25 shows the induction from somatic cells to primed pluripotent stem cells (left). Somatic cells were induced to naive stem cells, and then re-induced to primed pluripotent stem cells (right). Shown are photomicrographs of the cells, and the results of flow cytometry analysis after inducing differentiation of primordial germ cells.

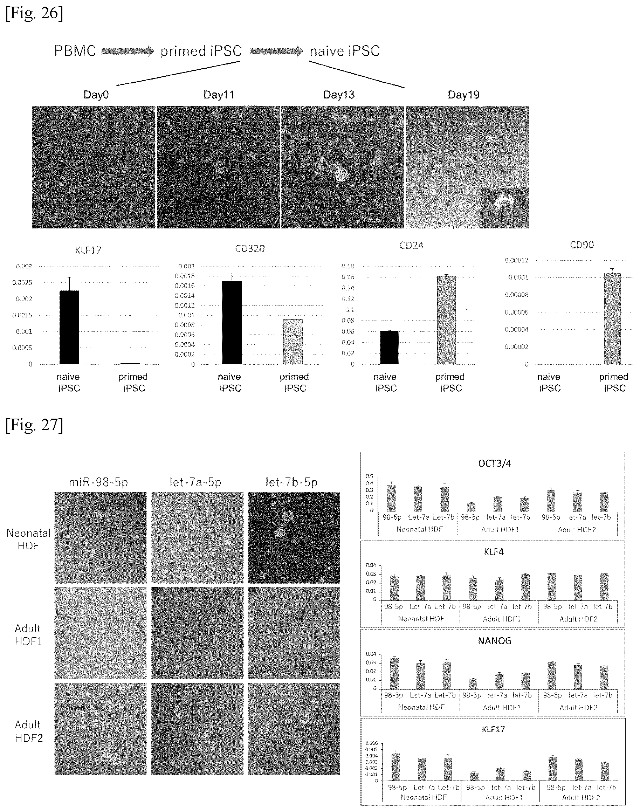

Fig. 26 shows the induction of somatic cells to primed iPS cells (primed iPSC) by an mRNA method, and induction to naive pluripotent stem cells by chemical resetting. Human peripheral blood mononuclear cells (PBMC) were used as the somatic cells. Phase contrast microscope photographs show the time-dependent change from the initial induction to naive pluripotent stem cells (Day 0), until typical naive pluripotent stem cell colonies were obtained (Day 13). The result of 19 days of culturing after the initial induction and analysis by qPCR indicated expression of genes expressed by naive pluripotent stem cells (bottom).
Fig. 27 shows the induction of naive pluripotent stem cells from somatic cells using the mRNA method. The somatic cells used were a neonatal fibroblast line (Neonatal HDF) and an adult fibroblast line (Adult HDF1 (Promocell), HDF2 (Thermo)), and the microRNAs used were miR-98-5p, let-7a-5p and let-7b-5p. Shown are the phase contrast microscope photographs of the obtained naive pluripotent stem cells (left) and the results of analyzing the expression levels of pluripotent stem cell marker genes (Oct3/4, KLF4, NANOG, KLF17) by qPCR (right).

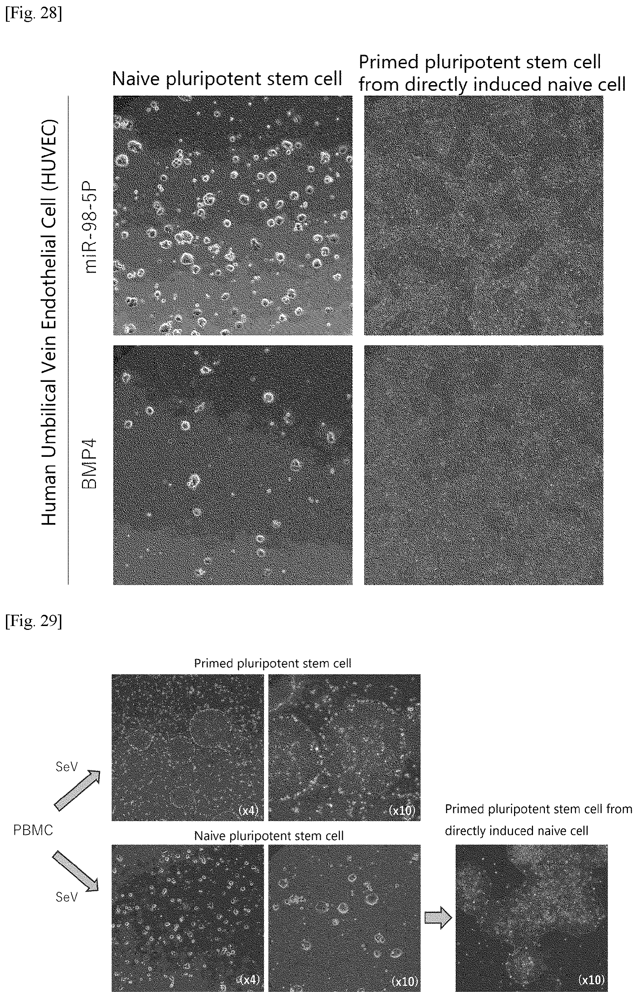

Fig. 28 shows the induction of naive pluripotent stem cells from somatic cells, and induction of primed pluripotent stem cells from the naive pluripotent stem cells (primed pluripotent stem cells from directly induced naive cells). Human umbilical vein endothelial cells (HUVEC) were used as the somatic cells, with microRNA (miR-98-5p) or BMP4 employed for induction to naive cells (left). These naive cells were then induced to primed pluripotent stem cells, to obtain primed pluripotent stem cells from directly induced naive cells (right). Phase contrast microscope photographs of each cell type are shown.
Fig. 29 shows the induction of primed or naive pluripotent stem cells from somatic cells using Sendai virus vector (SeV), and the induction of primed pluripotent stem cells from the naive pluripotent stem cells (primed pluripotent stem cells from directly induced naive cells). Human peripheral blood mononuclear cells (PBMC) were used as the somatic cells. Phase contrast microscope photographs of each cell type are shown.

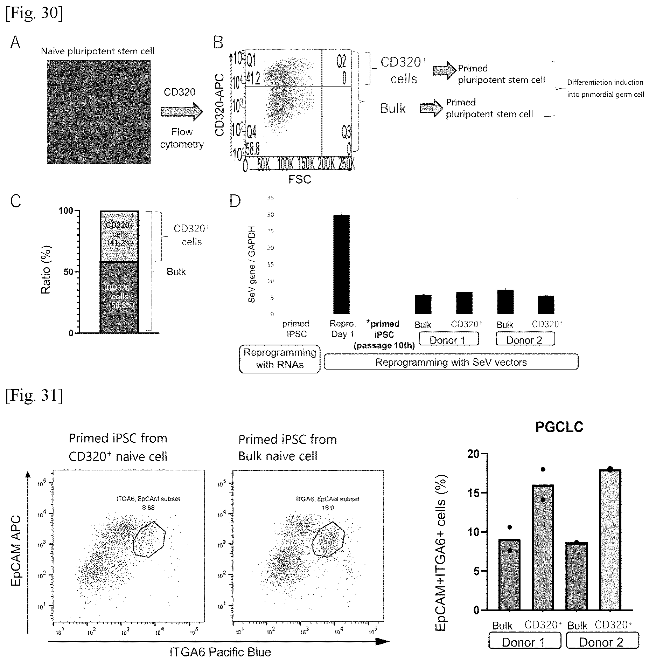

Fig. 30 shows sorting of CD320-high positive cells from the naive pluripotent stem cells (A) in Fig. 29 using flow cytometry, and induction of primed pluripotent stem cells from CD320-high positive cells (CD320+ cells) and unsorted cells (Bulk) (primed pluripotent stem cells from directly induced naive cells) (B). The proportions of CD320+ cells and CD320- cells among the unsorted cells are shown in C. The residual amount of Sendai virus genome measured by qPCR is shown in D. Sendai virus remained in both the CD320-high positive cells and the unsorted cells (Bulk). The primed iPSCs are primed iPSCs induced not by SeV but by the mRNA method (primed iPSC in Fig. 26). Day 1 represents iPSCs at Day 1 (the day following infection with SeV), induced to the naive type by the SeV method. *primed iPSC (Passage 10th) represents primed iPSCs induced by the SeV method after 10 passages (cells shown at the top in Fig. 29).
Fig. 31 shows the induction of differentiation of the primed pluripotent stem cells derived from CD320-high positive (CD320+ cells) or unsorted (Bulk) naive cells shown in Fig. 30 to primordial germ cells, and analysis of differentiation efficiency by flow cytometry. At left is shown a dot plot analyzing surface expression of two different primordial germ cell markers (CD326 (EpCAM) and CD49f (ITGA6)), with the polygon-enclosed region representing the double-positive cells (PGCLC). At right is shown a graph representing percentage of PGCLC (differentiation efficiency to PGCLC).

### DESCRIPTION OF EMBODIMENTS

### 1. Method for producing primed pluripotent stem cells, and primed pluripotent stem cells obtained by the method

The present invention provides a method for producing primed pluripotent stem cells with superior differentiation potency. Specifically, the present invention provides a method for producing primed pluripotent stem cells, comprising:
(1) a step of preparing primate naive pluripotent stem cells, and
(2) a step of inducing primed pluripotent stem cells from the naive pluripotent stem cells prepared in step (1) (hereinafter sometimes referred to as "production method of the present invention").

Unless otherwise specified, the pluripotent stem cells mentioned below are primate pluripotent stem cells. Such primate is not particularly limited and includes a haplorhine such as a tarsier, long-tailed macaque, cynomolgus monkey, rhesus monkey, Japanese macaque, gibbon, spider monkey, capuchin monkey, orangutan, gorilla, chimpanzee or human, or a strepsirrhine such as a lemur, aye-aye or loris, but a human is preferred.

The term "cells" as used herein includes "cell populations", unless otherwise specified. A cell population may be composed of a single type of cell or two or more different types of cells. A cell population may also take the form of a cell aggregate such as a spheroid or organoid.

The term "pluripotent stem cells" as used herein refers to stem cells that can differentiate into tissue or cells having various different forms or functions in the body, and that has the ability to differentiate into cells of lineages of the three germ layers (endoderm, mesoderm and ectoderm) (i.e. pluripotency). Examples of pluripotent stem cells to be used for the present invention include induced pluripotent stem cells (iPS cells, or iPSC), embryonic stem cells (ES cells), embryonic stem cells from cloned embryos obtainable by nuclear transfer (nuclear transfer embryonic stem cells, or ntES cells), and embryonic germ cells (EG cells), of which ES cells and iPS cells are preferred, and iPS cells (especially human iPS cells) are more preferred. When the pluripotent stem cells are ES cells or arbitrary cells derived from human embryos, the cells may be obtained by destruction of the embryo or they may be obtained without destruction of the embryo, but from an ethical standpoint they are preferably cells obtained without destruction of the embryo.

ES cells are stem cells having pluripotency and auto-replicating proliferation potency, established from the inner cell mass of an early embryo (such as the blastocyst) of a mammal such as a human or mouse. ES cells were discovered in mice in 1981 (M.J. Evans and M.H. Kaufman (1981), Nature 292:154-156), and ES cell lines were later established in primates including humans and monkeys (J.A. Thomson et al. (1998), Science 282:1145-1147; J.A. Thomson et al. (1995), Proc. Natl. Acad. Sci. USA, 92:7844-7848; J.A. Thomson et al. (1996), Biol. Reprod., 55:254-259; J.A. Thomson and V.S. Marshall (1998), Curr. Top. Dev. Biol., 38:133-165).

ntES cells are ES cells derived from a cloned embryo prepared by nuclear transfer technique, and they have approximately the same properties as fertilized egg-derived ES cells (Wakayama T. et al. (2001), Science, 292:740-743; S. Wakayama et al. (2005), Biol. Reprod., 72:932-936; Byrne J. et al. (2007), Nature, 450:497-502). In other words, ntES (nuclear transfer ES) cells are ES cells established from the inner cell mass of a cloned embryo-derived blastocyst obtained by replacing an unfertilized egg nucleus with a somatic cell nucleus.

iPS cells are cells obtainable by reprogramming of mammalian somatic cells or undifferentiated stem cells by introduction of specific factors (nuclear reprogramming factors). EG cells are cells with pluripotency similar to ES cells, being established from embryonic primordial germ cells.

As used herein, the term "naive pluripotent stem cells", also referred to as a "reset cells", means pluripotent stem cells having properties similar to a preimplantation embryo. Specifically, naive pluripotent stem cells have the following features, in addition to pluripotency and self-renewal ability.
(A) exhibiting dome-shaped colony formation, with smaller colony sizes compared to primed pluripotent stem cells.

Naive pluripotent stem cells typically also have the following features (B) and (C).
(B) Expressing one or more markers selected from the group consisting of CD320, SUSD2, CD75, KLF17 and TFCP2L1. Preferably at least CD320 is expressed, and more preferably CD320 and SUSD2 are expressed.
(C) Extensive DNA throughout the entire genome is demethylated.

The naive pluripotent stem cells used for the present invention may also further have either or both of the following features (D) and (E).
(D) In cells having a pair of XX sex chromosomes, both X chromosomes are activated (XaXa).
(E) Expressing one or more (and preferably all three) miRNAs selected from the group consisting of miR-373-3p, miR-520c-3p and miR-520h.

As used herein, the term "primed pluripotent stem cells" refers to pluripotent stem cells having properties similar to the post-implantation embryo epiblast, examples of which are common induced pluripotent stem cells or human ES cells obtainable by introducing reprogramming factors into somatic cells, as cells that have not been induced to the naive state as described below. Specifically, primed pluripotent stem cells have the following features, in addition to pluripotency and self-renewal ability.

(A') exhibiting flat colony formation, with larger colony sizes than naive pluripotent stem cells.

Primed pluripotent stem cells also typically have the following features (B') and (C').
(B') Not expressing CD320, SUSD2, CD75, KLF17 or TFCP2L1, and expressing CD24 and CD90.
(C') Higher degree of methylation of the genome than naive pluripotent stem cells.

The primed pluripotent stem cells used for the present invention may also further have either or both of the following features (D') and (E').
(D') In cells having a pair of XX sex chromosomes, one of the X chromosome is either inactivated (XaXi) or undergoing erosion (XeXi).
(E') Not expressing miR-520h.

That a protein or miRNA is "not expressed" or "negative", for the purpose of the present invention, means that no expression is found by the same methods used in the Examples described herein.

Step (1) of the production method of the present invention can be carried out by producing naive pluripotent stem cells from primed pluripotent stem cells by a publicly known method (also referred to as "naive-inducing treatment"). According to one aspect, therefore, step (1) of the production method of the present invention is a step of culturing primate primed pluripotent stem cells to produce naive pluripotent stem cells. Specific examples of such methods include a method of transiently overexpressing NANOG and KLF2 in primed pluripotent stem cells (NPL 1), a method of culturing primed pluripotent stem cells in the presence of 5 different kinase (MEK, GSK3, BRAF, SRC and ROCK) inhibitors, LIF and activin A (5i/L/A) (NPL 2), and a method of culturing primed pluripotent stem cells in the presence of an HDAC (histone deacetylase) inhibitor (NPL 3). Production may also be by the method illustrated in Fig. 1. Alternatively, the naive pluripotent stem cells to be used for the present invention may be obtained by purchasing, etc. an already established naive pluripotent stem cell line. The term "producing" pluripotent stem cells may also be interpreted as "establishing" or "inducing" pluripotent stem cells.

Examples of the maintenance medium for naive pluripotent stem cells include basal medium which further comprises one or more compounds selected from among STAT3 activators (typically LIF (Leukemia Inhibitory Factor)), MEK inhibitor, GSK3 inhibitor, cAMP production promoter, TGF-β inhibitor, PKC inhibitor and Tankyrase/Wnt/β-catenin inhibitor. Medium to be used for inducing naive cells may or may not include a STAT3 activator (typically LIF), depending on the pluripotent stem cell line. Medium for culturing naive pluripotent stem cells, on the other hand, can maintain the cells even without including a STAT3 activator (typically LIF). Typically, medium containing a MEK inhibitor is used, but preferably medium containing both a MEK inhibitor and LIF is used, and the basal medium used is N2B27 medium (marketed as Ndiff227 [Takara Bio, Cat. Y40002]) (1:1 mixture of N2 medium with N2 supplement added to DMEM/F12 medium and B27 medium with B27 supplement added to Neurobasal medium). The naive pluripotent stem cell maintenance medium also preferably comprises essentially no bFGF. According to one aspect, the naive pluripotent stem cell maintenance medium does not contain XAV939, and typically it also contains no Tankyrase/Wnt/β-catenin inhibitor other than XAV939. According to yet another aspect, the naive pluripotent stem cell maintenance medium does not contain CHIR99021, and typically it also contains no GSK3 inhibitor other than CHIR99021.

The STAT3 activator is not particularly limited, and examples include interleukin-6 (IL-6), Leukemia Inhibitory Factor (LIF), oncostatin M, leptin and Epidermal Growth Factor (EGF). IL-6 or LIF is preferred, with LIF being more preferred. The LIF referred to here is preferably human LIF. The LIF concentration in the medium is 1 ng/ml to 100 ng/ml or 5 ng/ml to 50 ng/ml, such as about 10 ng/ml.

MEK inhibitors are not particularly limited, and examples include PD0325901 (N-[(2R)-2,3-dihydroxypropoxy]-3,4-difluoro-2-[(2-fluoro-4-iodophenyl)amino]-benzamide; CAS Accession No.: 391210-10-9), U0126 (1,4-diamino-2,3-dicyano-1,4-bis[2-aminophenylthio]butadiene; CAS Accession No.: 109511-58-2), PD98059 (2-(2-amino-3-methoxyphenyl)-4H-1-benzopyran-4-one; CAS Accession No.: 167869-21-8), and PD184352 (2-(2-chloro-4-iodophenylamino)-N-cyclopropylmethoxy-3,4-difluorobenzamide; CAS Accession No.: 212631-79-3. PD0325901 is preferred among these. When PD0325901 is used as a MEK inhibitor, its concentration in the medium may be 50 nM to 100 µM or 100 nM to 10 µM, such as about 1 µM.

The GSK3 inhibitor is not particularly limited, and examples include CHIR99021 (6-[[2-[[4-(2,4-dichlorophenyl)-5-(5-methyl-1H-imidazol-2-yl)-2-pyrimidinyl]amino]ethyl]amino]-3-pyridinecarbonitrile; CAS Accession No.: 252917-06-9), BIO (6-bromoindirubin-3'-oxime; CAS Accession No.: 667463-62-9), Kenpaullone (9-bromo-7,12-dihydroindolo[3,2-d][1]benzazepine-6(5H)-one; CAS Accession No.: 142273-20-9) and IM-16 (3-(4-fluorophenylethylamino)-1-methyl-4-(2-methyl-1H-indol-3-yl)-1H-pyrrole-2,5-dione; CAS Accession No.: 1129669-05-1). CHIR99021 is preferred among these. When CHIR99021 is used as a GSK3 inhibitor, its concentration in the medium may be 50 nM to 100 µM or 100 nM to 10 µM, such as about 1 µM.

The cAMP production promoter is not particularly limited, and forskolin is one example. When forskolin is used as the cAMP production promoter, its concentration in the medium may be 50 nM to 100 µM or 100 nM to 10 µM, such as about 1 µM.

The TGF-β inhibitor is not particularly limited, and examples include A83-01 (3-(6-methyl-2-pyridinyl)-N-phenyl-4-(4-quinolinyl)-1H-pyrazole-1-carbothioamide) and SB431542 (4-[4-(1,3-benzodioxol-5-yl)-5-(2-pyridinyl)-1H-imidazol-2-yl]-benzamide). A83-01 is preferred among these. When A83-01 is used as a TGF-β inhibitor, its concentration in the medium may be 10 nM to 100 µM or 100 nM to 10 µM, such as about 1 µM.

The PKC inhibitor is not particularly limited, and examples include Go6983 (3-[1-[3-(dimethylamino)propyl]-5-methoxy-1H-indol-3-yl]-4-(1H-indol-3-yl)-1H-pyrrole-2,5-dione; CAS Accession No.: 133053-19-7) and GF109203X (3-(1-(3-dimethylamino)propyl)-1H-indol-3-yl)-4-(1H-indol-3-yl)-1H-pyrrole-2,5-dione; CAS Accession No.: 133052-90-1). Go6983 is preferred among these. When Go6983 is used as an PKC inhibitor, its concentration in the medium may be 50 nM to 100 µM or 100 nM to 10 µM, such as about 1 µM to 3 µM.

The Tankyrase/Wnt/β-catenin inhibitor is not particularly limited, and examples include XAV939 (2-(4-trifluoromethylphenyl)-7,8-dihydro-5H-thiopyrano[4,3-d]pyrimidine-4(3H)-one; CAS Accession No.: 284028-89-3), IWT-2, IWP-3 and IWP-4. When XAV939 is used as a Tankyrase/Wnt/β-catenin inhibitor, its concentration in the medium may be 0.5 µM to 100 µM, such as about 2 µM.

The naive pluripotent stem cell maintenance medium may also contain an HDAC inhibitor. Examples of HDAC inhibitors include, but are not limited to, valproic acid (alternate name: 2-propylvaleric acid), sodium butyrate, suberoylanilide hydroxamic acid, BRD4884 (N-(4-amino-4'-fluoro[1,1'-biphenyl]-3-yl)tetrahydro-2H-pyran-4-carboxamide; CAS Accession No.: 1404559-91-6) and BRD6688 (N-[2-amino-5-(4-pyridinyl)phenyl]-1-pyrrolidinecarboxamide; CAS Accession No.: 1404562-17-9). The concentration of the HDAC inhibitor in the medium may be about 0.1 mM to 10 mM, and preferably about 0.5 mM to 5 mM.

A ROCK inhibitor may also be present in the medium. ROCK inhibitors are not particularly limited so long as they can inhibit the function of Rho-kinase (ROCK), and examples include Y-27632 (for example, see Ishizaki et al., Mol. Pharmacol. 57, 976-983(2000); Narumiya et al., Methods Enzymol. 325, 273-284(2000)), Fasudil/HA1077 (for example, see Uenata et al., Nature 389:990-994(1997)), H-1152 (for example, see Sasaki et al., Pharmacol. Ther. 93:225-232(2002)), Wf-536 (for example, see Nakajima et al., Cancer Chemother. Pharmacol. 52(4):319-324(2003)) and their derivatives, as well as antisense nucleic acid, RNA interference inducible nucleic acid (e.g., siRNA) and dominant negative mutants for ROCK, and their expression vectors. Other publicly known low molecular compounds may also be used as ROCK inhibitors (for example, see U.S. Patent Application Publication No. 2005/0209261, No. 2005/0192304, No. 2004/0014755, No. 2004/0002508, No. 2004/0002507, No. 2003/0125344, No. 2003/0087919, and International Patent Publication No. WO2003/062227, No. 2003/059913, No. 2003/062225, No. 2002/076976 and No. 2004/039796). The concentration of the ROCK inhibitor may be selected as appropriate by a person skilled in the art depending on the ROCK inhibitor used, and when Y-27632 is used as the ROCK inhibitor, for example, it may be 0.1 µM to 100 µM, preferably 1 µM to 50 µM and more preferably 5 µM to 20 µM.

The following may be specifically mentioned as maintenance media for naive pluripotent stem cells.
· t2iLGo (NPL 1)
   N2B27 + PD0325901 (1 µM) + CHIR99021 (1 µM) + LIF (10 ng/ml) + Go6983 (2-3 µM)
· 5i/L/A (NPL 2)
   N2B27 + PD0325901 (1 µM) + CHIR99021 (1 µM) + SB590885 (0.5 µM) + WH-4-023 (1 µM) + Y-27632 (10 µM) + LIF (10 ng/ml) + Activin A (20 ng/ml)
· tt2iLc + iWNT (NPL 3)
   N2B27 + PD0325901 (1 µM) + LIF (10 ng/ml) + Go6983 (2 µM) + XAV939 (2 µM)
· Chemical Resetting Medium 1 (cRM-1)
   N2B27 + PD0325901 (1 µM) + LIF (10 ng/ml) + sodium valproate salt (1 mM)
· Chemical Resetting Medium 2 (cRM-2)
   N2B27 + PD0325901 (1 µM) + LIF (10 ng/ml) + Go6983 (2 µM) + XAV939 (2 µM)

The maintenance medium for naive pluripotent stem cells and the maintenance medium for primed pluripotent stem cells can typically be distinguished in that the maintenance medium for primed pluripotent stem cells comprises essentially no LIF while the culture medium for naive pluripotent stem cells comprises essentially no bFGF.

The maintenance medium for naive pluripotent stem cells may be appropriately exchanged during the culturing, and for example, cRM-1 may be used for culturing from day 1 to day 4, with cRM-2 being used for culturing thereafter.

A commercially available naive pluripotent stem cell maintenance medium, such as NaiveCult Induction Kit [STEMCELL Technologies, Cat. ST-05580] or NaiveCult Expansion Medium [STEMCELL Technologies, Cat. ST-05590], may also be used.

The period for inducing naive pluripotent stem cells from primed pluripotent stem cells is not particularly limited, and will usually be for 6 days or longer (such as 7 days, 8 days, 9 days, 10 days, or longer). Since the proportion of cells expressing CD320 by day 14 from the start of culturing of the primed pluripotent stem cells was found to increase as demonstrated in the Examples below, the induction period is preferably 14 days or longer and more preferably 19 days or longer (such as 20 days, or even longer). The upper limit is not particularly limited but will typically be 70 days or less (20 passages or less).

The step of inducing naive pluripotent stem cells from primed pluripotent stem cells may also include a step of sorting out (in other words, isolating or purifying) from among the cell population the cells positive for markers expressed by naive pluripotent stem cells. Examples of such markers include CD320, SUSD2 and CD75, with CD320 being especially preferred. As demonstrated in the Examples below, CD320 can serve as a marker for naive pluripotent stem cells in the more undifferentiated state, and therefore sorting out CD320-positive cells (especially CD320-high positive cells) allows naive pluripotent stem cells in a more undifferentiated state to be concentrated. Naive pluripotent stem cells can also be sorted out based on X chromosome activation or other indicators, in addition to markers expressed by naive pluripotent stem cells.

As used herein, "CD320-positive" means "produces protein encoded by CD320 mRNA", unless otherwise specified. Cells may therefore be said to be CD320-positive if CD320 protein is detected by the method described in the Examples below (flow cytometry). This also applies to markers expressed by naive pluripotent stem cells, other than CD320. The term "positive" as used herein includes the concept of high expression (also referred to "high positive") and weak expression (also referred to "low positive"). High positivity and low positivity can be judged based on a chart obtained by flow cytometry. The position in the chart may vary depending on the voltage setting and sensitivity setting of the device, and the antibody clone used, the staining conditions and the dye used, but a person skilled in the art can appropriately draw lines so as not to separate cell populations that are to be treated as a single group in the obtained chart.

The method of sorting the CD320-positive cells may be any appropriate known method, such as a method of labeling with an antibody for CD320, and using flow cytometry or mass cytometry, a magnetic cell separation method, or a method using an affinity column, etc. immobilizing a desired antigen.

The primed pluripotent stem cells as the source for the naive pluripotent stem cells may be produced by a publicly known method. For example, ES cells can be established by extracting the inner cell mass from the blastocyst of a fertilized egg of a target animal and culturing the inner cell mass on a fibroblast feeder. Alternatively, ES cells can be established using a single embryo blastomere alone during the cleavage stage before the blastocyst stage (Chung Y. et al. (2008), Cell Stem Cell 2: 113-117), or they can be established using a developmentally arrested embryo (Zhang X. et al. (2006), Stem Cells 24: 2669-2676.).

Combinations of nuclear transfer (Cibelli J.B. et al. (1998), Nature Biotechnol., 16:642-646) and ES cell preparation techniques (described above) are used to prepare ntES cells (Wakayama, S. (2008), Jikken Igaku, Vol.26, No.5 (special edition), pp.47-52). For nuclear transfer, a somatic cell nucleus is injected into a mammalian enucleated unfertilized egg and cultured for several hours, thereby enabling the reprogramming of the somatic cell.

The ES cell line used for the present invention may be, for human ES cell lines, any of the different human ES cell lines established by the University of Wisconsin, NIH, Riken, Kyoto University, the National Center for Child Health and Development or Cellartis, for example. Specific examples of human ES cell lines include CHB-1 to CHB-12, RUES1, RUES2 and HUES1 to HUES28 distributed by ESI Bio Co., H1 and H9 distributed by WiCell Research, and KhES-1, KhES-2, KhES-3, KhES-4, KhES-5, SSES1, SSES2 and SSES3 distributed by Riken.

A large number of iPS cells currently exist, among which there may be used human iPSCs established by introduction of the 4 factors Oct3/4·Sox2·Klf4·c-Myc into human fibroblasts by Yamanaka et al. (Takahashi K, Yamanaka S., et al. Cell, (2007) 131:861-872.), Nanog-iPSCs established by sorting based on Nanog expression markers after introduction of the 4 factors (Okita, K., Ichisaka, T. and Yamanaka, S. (2007). Nature 448, 313-317.), iPSCs produced by methods without c-Myc (Nakagawa M, Yamanaka S., et al. Nature Biotechnology, (2008) 26, 101-106), and iPSCs established by introduction of 6 factors by a virus-free method (Okita K et al. Nat. Methods 2011 May; 8(5):409-12, Okita K et al. Stem Cells. 31(3):458-66.), etc. The induced pluripotent stem cells established by introduction of the 4 factors OCT3/4·SOX2·NANOG·LIN28, created by Thomson et al. (Yu J., Thomson JA. et al., Science (2007) 318: 1917-1920), the induced pluripotent stem cells created by Daley et al. (Park IH, Daley GQ. et al., Nature (2007) 451:141-146) and the induced pluripotent stem cells created by Sakurata et al. (Japanese Unexamined Patent Publication No. 2008-307007), etc. may also be used.

In addition, any induced pluripotent stem cells publicly known in the field as described in published literature (for example, Shi Y., Ding S., et al., Cell Stem Cell, (2008) Vol.3, Issue 5, 568-574;, Kim JB., Scholer HR., et al., Nature, (2008) 454, 646-650; Huangfu D., Melton, DA., et al., Nature Biotechnology, (2008) 26, No 7, 795-797), or patents (for example, Japanese Unexamined Patent Publication No. 2008-307007, Japanese Unexamined Patent Publication No. 2008-283972, US2008-2336610, US2009-047263, WO2007-069666, WO2008-118220, WO2008-124133, WO2008-151058, WO2009-006930, WO2009-006997 and WO2009-007852), may also be used. Factors that contribute to reprogramming of somatic cells, such as Oct3/4, Sox2, Klf4, c-Myc, NANOG and LIN28, are "reprogramming factors".

Induced pluripotent stem cell lines that are iPSC lines established by NIH, Riken or Kyoto University, for example, may be used as well. Examples include human iPSC lines such as HiPS-RIKEN-1A, HiPS-RIKEN-2A, HiPS-RIKEN-12A and Nips-B2 by Riken, and AdiPS cells, 253G1, 253G4, 1201C1, 1205D1, 1210B2, 1383D2, 1383D6, 201B7, 409B2, 454E2, 585A1, 585B2, 606A1, 610B1, 648A1, 1231A3 and FfI-01s04 by Kyoto University.

The induced pluripotent stem cells to be used in the production method of the present invention may be cells from a patient with a hereditary disease. Cells to be induced to differentiate from pluripotent stem cells from a patient with a hereditary disease can serve as a disease model reflecting pathology of the patient, and are therefore suitable for screening of therapeutic or prophylactic medicaments for the patient, etc. Alternatively, pluripotent stem cells from a patient with a hereditary disease may be differentiated to cells or an organoid of interest after repair of a gene by genome editing using a CRISPR-Cas system, etc., using the cells as a therapeutic agent for the disease.

EG cells can be established by culturing primordial germ cells in the presence of substances such as LIF, bFGF and stem cell factors (Matsui Y. et al. (1992), Cell, 70:841-847; J. L. Resnick et al. (1992), Nature, 359:550-551). Other pluripotent stem cells can also be established by publicly known methods.

The naive pluripotent stem cells prepared in step (1) of the production method of the present invention may be cells produced from somatic cells without transitioning through primed pluripotent stem cells. As used herein, "produced without transitioning through primed pluripotent stem cells" means that the method is not one in which somatic cells are induced after culturing in maintenance medium for primed pluripotent stem cells as defined above after having introduced a reprogramming factor into the cells, which however does not exclude cases where primed pluripotent stem cells or cells similar to primed pluripotent stem cells are transiently produced during production of naive pluripotent stem cells. The phrase "cells similar to primed pluripotent stem cells" means cells that are not multipotent but express one or more pluripotent stem cell markers selected from the group consisting of Nanog, Oct4, TRA-1-60 and SSEA-3. The phrase "cultured in maintenance medium for primed pluripotent stem cells" means comprising a step of culturing the cells in medium containing bFGF from a certain period of time at or after introduction of a reprogramming factor (typically 12 hours to 2 days after final introduction of the reprogramming factor). The primed pluripotent stem cells obtainable by induction from naive pluripotent stem cells produced in this manner are also referred to as "primed pluripotent stem cells from directly induced naive cells".

Reprimed pluripotent stem cells and primed pluripotent stem cells from directly induced naive cells may collectively be referred to as "pluripotent stem cells of the present invention" to include both primed pluripotent stem cell types, unless otherwise specified. Thus, in another aspect, primed pluripotent stem cells obtained by the production method of the present invention (i.e. pluripotent stem cells of the present invention) are provided.

Reprimed pluripotent stem cells and primed pluripotent stem cells from directly induced naive cells are conjectured to have equivalent genomic methylation states and differentiation potency. Therefore, the pluripotent stem cells of the present invention may be cells with the aforementioned features of primed pluripotent stem cells (except for features (C) and (C')), as well as having high differentiation potency and a low degree of genomic methylation. The pluripotent stem cells of the present invention may therefore be considered to be "primed pluripotent stem cells having high differentiation potency". The primed pluripotent stem cells having high differentiation potency are typically cells in which "genomic methylation has been reset once."

As used herein, "high differentiation potency" (in other words, "low differentiation resistance") means that, for reprimed pluripotent stem cells, "differentiation potency is higher" (in other words, "differentiation resistance is lower") compared to primed pluripotent stem cells before naive-inducing treatment. For primed pluripotent stem cells from directly induced naive cells, it means that "differentiation potency is higher" (in other words, "differentiation resistance is lower") compared to primed pluripotent stem cells that have been induced from the same somatic cell line without transitioning through naive pluripotent stem cells. The term "high differentiation potency" means high differentiation efficiency to at least one type (normally multiple types) of cells.

As used herein, the term "somatic cells" refers to cells other than germ cells, among the cells of an animal. Somatic cells include, but are not particularly limited to, fetal somatic cells, neonatal somatic cells, and mature healthy or diseased somatic cells, and also include primary cultured cells, subcultured cells and established cell lines. Specific examples of somatic cells include (1) tissue stem cells (somatic stem cells) such as neural stem cells, hematopoietic progenitor cells, mesenchymal stem cells and dental pulp stem cells, (2) tissue progenitor cells and (3) differentiated cells such as lymphocytes, epithelial cells, endothelial cells, muscle cells, fibroblasts (including skin cells), hair cells, hepatocytes, gastric mucosal cells, enterocytes, splenocytes, pancreatic cells (including exocrine pancreatic cells), brain cells, lung cells, renal cells and adipocytes.

Directly induced primed pluripotent stem cells can be produced, for example, by introducing a reprogramming factor and miRNA belonging to the let-7-5p miRNA family into somatic cells and culturing the cells in the maintenance medium for naive pluripotent stem cells. According to one aspect of the present invention, therefore, step (1) of the production method of the present invention is a step in which somatic cells into which a reprogramming factor and miRNA belonging to the let-7-5p miRNA family have been introduced are cultured in the maintenance medium for naive pluripotent stem cells, the step typically not including a step of culturing cells in maintenance medium for primed pluripotent stem cells. When miRNA belonging to the let-7-5p miRNA family is added, and/or expression of the target protein of the miRNA is suppressed, it is possible to accelerate production of directly induced primed pluripotent stem cells.

Somatic cells into which a reprogramming factor and miRNA belonging to the let-7-5p miRNA family have been introduced may be prepared by introducing a reprogramming factor and miRNA belonging to the let-7-5p miRNA family into somatic cells simultaneously, or prepared by introducing miRNA belonging to the let-7-5p miRNA family after elapse of a period into somatic cells which have previously had a reprogramming factor inserted, or prepared by introducing a reprogramming factor after elapse of a period into somatic cells which have previously had let-7-5p miRNA family miRNA introduced. Introduction of a reprogramming factor and/or miRNA belonging to the let-7-5p miRNA family into somatic cells may be carried out once or several times (such as 2, 3, 4, 5 or more times).

According to one aspect, the naive pluripotent stem cells prepared in step (1) of the production method of the present invention may be produced by introducing miRNA belonging to the let-7-5p miRNA family into somatic cells, and/or by suppressing expression of the target protein of the miRNA. Step (1) of the production method of the present invention may be a step of culturing cells that have had miRNA belonging to the let-7-5p miRNA family introduced and/or that have expression of a target protein of the miRNA suppressed, to produce naive pluripotent stem cells.

Therefore, in another aspect of the present invention, a method for producing naive pluripotent stem cells comprising a step in which somatic cells into which a reprogramming factor and miRNA belonging to the let-7-5p miRNA family have been introduced are cultured in the maintenance medium for naive pluripotent stem cells (this may hereinafter be referred to as "direct method of inducing naive pluripotent stem cells of the present invention") is provided.

Examples of miRNA belonging to the let-7-5p miRNA family include miR-98-5p, let-7a-5p, let-7b-5p, let-7c-5p, let-7e-5p, let-7f-5p, let-7g-5p and let-7i-5p. The sequences of these miRNA are shown in Table 1. Similar sequences to these, such as (1) miRNA consisting of a sequence having at least 90% sequence identity, and preferably at least 95% (such as 96%, 97%, 98%, 99% or greater) sequence identity with the sequences listed in Table 1, (2) miRNA consisting of a sequence which is a sequence listed in Table 1 having a deletion, substitution, insertion and/or addition of one or more (such as 2 or 3) bases, and (3) orthologs of the sequences listed in Table 1 in a mammal other than a human (preferably a primate), may also be used, since they can retain the same function as miRNA belonging to the let-7-5p miRNA family. One of these may be used alone, or two or more may be used in combination. Throughout the present specification, the "miRNA belonging to the let-7-5p miRNA family" includes miRNA having similar sequences to the sequences of miRNA listed in Table 1, as well as their mimics, unless otherwise specified.

**[Table 1]**

| Sequence name | Sequence | SEQ ID NO |
|---|---|---|
| miR-98-5p | UGAGGUAGUAAGUUGUAUUGUU | 1 |
| let-7a-5p | UGAGGUAGUAGGUUGUAUAGUU | 2 |
| let-7b-5p | UGAGGUAGUAGGUUGUGUGGUU | 3 |
| let-7c-5p | UGAGGUAGUAGGUUGUAUGGUU | 4 |
| let-7e-5p | UGAGGUAGGAGGUUGUAUAGUU | 5 |
| let-7f-5p | UGAGGUAGUAGAUUGUAUAGUU | 6 |
| let-7g-5p | UGAGGUAGUAGUUUGUACAGUU | 7 |
| let-7i-5p | UGAGGUAGUAGUUUGUGCUGUU | 8 |

The reprogramming factor to be introduced into the somatic cells may be in the form of protein, or it may be in the form of nucleic acid (RNA or DNA) coding for the protein or an expression vector containing the nucleic acid (for example, a viral vector such as Sendai virus, etc.). When the reprogramming factor is to be introduced in the form of RNA, the cell defense mechanism can potentially be activated by the immunogenic RNA introduced into the cells, and therefore RNA that avoids this defense mechanism (for example, vaccinia virus E3 mRNA, K3L mRNA, B18 mRNA, combinations of these mRNA, etc.) is preferably introduced into the somatic cells. For improved establishment efficiency of the pluripotent stem cells, each miRNA or its mimic (for example, miR-302a-3p or its mimic, miR-302b-3p or its mimic, miR-302c-3p or its mimic, miR-302d-3p or its mimic, miR-367-3p or its mimic, combinations of these miRNAs or their mimics, etc.) may be introduced into the somatic cells.

When miRNA such as miRNA belonging to the let-7-5p miRNA family is to be introduced into somatic cells, it may be in the form of naturally-occurring miRNA, or miRNA with chemical modification of the nucleic acid, or a miRNA mimic, or DNA coding for the miRNA or an expression vector containing the DNA, but it is preferably a miRNA mimic. A miRNA mimic may be in the form of double stranded RNA, and will typically be composed of a guide strand consisting of naturally-occurring RNA and a chemically modified (for example, 2'-O-methyl-modified) passenger strand, the guide strand exhibiting RNAi activity and the passenger strand not exhibiting RNAi activity, and it can mimic thus a naturally-occurring miRNA in cells.

Examples of target proteins of the miRNA belonging to the let-7-5p miRNA family to be used for the present invention include NR6A1 (Nuclear Receptor Subfamily 6 Group A Member 1), TRIM71 (Tripartite Motif Containing 71), IGF2BP1 (Insulin-like growth factor 2 mRNA-binding protein1), VASH2 (Vasohibin-2) or DPH3 (Diphthamide Biosynthesis 3), for example. It is most preferred to suppress the activity of NR6A1 and/or TRIM71. Of these, a single protein may be the target, or two or more different proteins may be targets.

The method for suppressing activity of the target protein of the miRNA belonging to the let-7-5p miRNA family is not particularly limited and may be a method of suppressing expression of the protein by a knockdown method, or a method of modifying the gene coding for a protein, by a knockout method. Examples of knockdown methods include methods of introducing siRNA, shRNA or antisense nucleic acid, and methods of introducing DNA coding for shRNA or antisense nucleic acid, into the cells. The DNA used is preferably an expression vector such as a plasmid. There may also be used an expression vector coding for shRNA or antisense nucleic acid whose expression can be induced by a drug. Examples of knockout methods include methods using genome editing technology with a CRISPR/Cas9 system, for example.

The timing and period for introducing miRNA belonging to the let-7-5p miRNA family into cells, expressing miRNA in cells and/or suppressing activity of target protein of the miRNA, is not particularly limited but is preferably at least 1 day, more preferably at least 3 days and even more preferably at least 5 days after introducing the reprogramming factor into the cells. While the upper limit is not particularly limited, it will typically be within the period in which naive pluripotent stem cells are obtainable (such as 19 days).

Step (1) in the production method of the present invention and the direct method of inducing naive pluripotent stem cells of the present invention may also include a step of further introducing a bone morphogenetic protein or a nucleic acid coding for the protein into the cells in which the miRNA belonging to the let-7-5p miRNA family has been introduced, and/or a step of culturing the cells into which the miRNA belonging to the let-7-5p miRNA family has been introduced, in medium containing a bone morphogenetic protein. The phrase "step of introducing a bone morphogenetic protein" may hereinafter be used as encompassing these steps.

Alternatively, naive pluripotent stem cells may be directly induced from somatic cells using a bone morphogenetic protein, without using miRNA belonging to the let-7-5p miRNA family. Yet another aspect of the present invention therefore provides a method for producing naive pluripotent stem cells, comprising a step of culturing somatic cells into which reprogramming factor and a bone morphogenetic protein have been introduced, in the maintenance medium for naive pluripotent stem cells. Specifically, such step can be carried out, for example, by a step of introducing a bone morphogenetic protein or nucleic acid coding for the protein, a step of culturing the somatic cells in medium containing the bone morphogenetic protein, and so on. Both of these steps are encompassed by the phrase "step of introducing a bone morphogenetic protein".

The somatic cells into which the reprogramming factor and bone morphogenetic protein have been introduced may be prepared by simultaneously introducing the reprogramming factor and bone morphogenetic protein into the somatic cells, or they may be prepared by introducing a bone morphogenetic protein after elapse of a period into somatic cells which have previously had a reprogramming factor inserted, or they may be prepared by introducing a reprogramming factor after elapse of a period into somatic cells which have previously had a bone morphogenetic protein introduced. Introduction of a reprogramming factor and/or bone morphogenetic protein into somatic cells may be carried out once or several times (such as 2, 3, 4, 5 or more times).

Bone morphogenetic proteins to be used for the present invention include, but are not limited to, BMP2, BMP4 and their analogs or derivatives. Human bone morphogenetic protein is a preferred bone morphogenetic protein. The bone morphogenetic protein added to the medium may also be a recombinant protein. When the bone morphogenetic protein is to be added to medium, the concentration of the protein in the medium will typically be 5 ng/mL to 100 ng/mL, and preferably 5 ng/mL to 10 ng/mL.

The methods of introducing the bone morphogenetic protein include a method of directly transfecting the cells with a synthetic protein molecule, a method of introducing mRNA coding for bone morphogenetic protein into the cells in the form of a synthetic RNA molecule, a method of introducing DNA coding for bone morphogenetic protein into the cells. A method of directly transfecting cells with a synthetic protein molecule may employ any desired transfection reagent.

Furthermore, a step of activating bone morphogenetic protein in cells may also be carried out, either instead of or in addition to the step of introducing the bone morphogenetic protein. Such a step will typically be carried out by adding a bone morphogenetic protein activator to the medium. When a bone morphogenetic protein activator is to be added to the medium, the concentration of the activator in the medium will typically be 7 µM to 50 µM, although this will depend on the type of activator.

The bone morphogenetic protein activator may be a substance that increases the activity of cell-intrinsic bone morphogenetic protein, or it may be a substance that increases the activity of the bone morphogenetic protein that has been externally added in the step of introducing a bone morphogenetic protein. Examples of such bone morphogenetic protein activators include, but are not limited to, SB-4 (CAS Accession No.: 100874-08-6) and isoliquiritigenin (CAS Accession No.: 961-29-5).

The timing and period for introducing the bone morphogenetic protein into cells, expressing the protein in cells and/or activating the protein, is not particularly limited, but is preferably at least 1 day, more preferably at least 3 days and even more preferably at least 5 days after introducing the reprogramming factor into the cells, for example. While the upper limit is not particularly limited, it will typically be within the period in which naive pluripotent stem cells are obtainable (such as 19 days). Another preferred mode is to add the bone morphogenetic protein to the medium 1 day after introducing the reprogramming factor into the cells (with addition being at a concentration of 5 ng/mL to 10 ng/mL, for example).

Examples of expression vectors include viral vectors such as retroviruses, lentiviruses, adenoviruses, adeno-associated viruses, herpesviruses and Sendai viruses, as well as plasmid vectors, episomal vectors, artificial chromosome vectors and transposon vectors (piggyBac, piggyBat, TolII). The promoter used for an expression vector, for example, may be EF1α promoter, ACTB promoter, UbqC promoter, PGK promoter, CAG promoter, SRα promoter, SV40 promoter, LTR promoter, CMV (cytomegalovirus) promoter, RSV (Rous sarcoma virus) promoter, MoMuLV (Moloney murine leukemia virus) LTR, HIV LTR, and HSV-TK (herpes simplex virus thymidine kinase) promoter. On the other hand, preferred promoters for expression vectors containing DNA coding for miRNA, shRNA or antisense nucleic acid are pol III promoters (such as SNR6, SNR52, SCR1, RPR1, U3, U6 and H1 promoter).

RNA such as mRNA and miRNA may be produced by chemical synthesis, or they may be produced by *in vitro* transcription (IVT) method. Alternatively, a product of expression in an organism *(E. coli* or mammalian cultured cells) may be purified. The chemical synthesis method may employ nucleoside phosphoroamidite and a solid phase carrier, for example. By using nucleoside phosphoroamidite with chemical modification (such as sugar 2'-O-methylated phosphoroamidite), it is possible to introduce a modified nucleotide into any nucleotide position of an RNA sequence.

The introduction of the nucleic acid (such as mRNA or miRNA, or DNA coding for them), expression vector containing the nucleic acid, or protein (such as reprogramming factor) into the cells may be carried out by any of various publicly known methods. Examples of such methods include calcium phosphate-mediated transfection, electroporation, liposome transfection, lipofection, gene gun, microinjection, viral vector methods, virus-like particle methods, *Agrobacterium* methods, agroinfiltration methods, PEG-calcium methods, sonoporation methods and lipid nanoparticle methods.

The period for introducing the reprogramming factor into somatic cells to produce naive pluripotent stem cells is not particularly limited, but will usually be 9 days or longer, preferably 14 days or greater, more preferably 19 days or longer and even more preferably 25 days or longer. The upper limit is not particularly limited but will typically be 70 days or less (about 20 passages).

Step (2) of the production method of the present invention can be carried out by culturing naive pluripotent stem cells in maintenance medium for primed pluripotent stem cells. The medium is not limited so long as it allows maintenance culturing of primed pluripotent stem cells, but it will typically be medium containing bFGF. Medium containing bFGF can typically be prepared by adding bFGF to the basal medium specified below. In this way, primed pluripotent stem cells that are reproduced from naive pluripotent stem cells may be referred to as "reprimed (or re-primed) pluripotent stem cells" to distinguish them from primed pluripotent stem cells as starting cells for naive pluripotent stem cells.

The culturing period for step (2) of the production method of the present invention is not particularly limited but will typically be 4 days or longer (such as 5 days, 6 days, 7 days, 8 days, 9 days or 10 days or longer). Reprimed pluripotent stem cells can undergo maintenance proliferation in medium containing bFGF, and while the upper limit is not particularly limited it will typically be 40 days or less and preferably 30 days or less.

In the production method of the present invention, the culturing may be carried out under feeder-free conditions and/or xeno-free conditions, for all or part of the culturing period. From the viewpoint of use in the clinic, the culturing in the production method of the present invention is preferably carried out under feeder-free and xeno-free conditions during the entire period. For the present purpose, "feeder-free" means medium or culturing conditions without other cell types (i.e., feeder cells) with an assisting role that are used to prepare culturing conditions for the cells to be cultured. The term "xeno-free" means medium or culturing conditions without components derived from a different organism than the species of the cells to be cultured.

The basal media to be used in the production method of the present invention is not particularly limited and may include, for example, StemFit (registered trademark)AK02 medium (Ajinomoto Co., Inc.), StemFit (registered trademark) AK03 medium (Ajinomoto Co., Inc.), StemFit (registered trademark) Basic03 medium, CTS (registered trademark) KnockOut SR XenoFree Medium (Gibco), mTeSR1 medium, TeSR1 medium (Stem Cell Technologies), Iscove's modified Dulbecco's medium (GE Healthcare) or Improved MEM (Thermo Fisher Scientific). These media can be used for culturing under feeder-free and xeno-free conditions. Additional media include, but are not limited to, RPMI-1640 medium, Eagle MEM (EMEM), Dulbecco's modified MEM, Glasgow's MEM (GMEM), α-MEM, 199 medium, IMDM, DMEM, Hybridoma Serum free medium, KnockOut^{™} DMEM, Advanced TM medium (e.g.: Advanced MEM, Advanced RPMI and Advanced DMEM/F-12), Ham's Medium F-12, Ham's Medium F-10, Ham's Medium F12K, DMEM/F-12, ATCC-CRCM30, DM-160, DM-201, BME, Fischer, McCoy's 5A, Leibovitz's L-15, RITC80-7, MCDB105, MCDB107, MCDB131, MCDB153, MCDB201, NCTC109, NCTC135, Waymouth's Medium (e.g.:Waymouth's MB752/1), CMRL medium (e.g.: CMRL-1066), Williams' medium E, Brinster's BMOC-3 Medium, E8 Medium, StemPro 34, MesenPRO RS (all by Thermo Fisher Scientific), ReproFF2, Primate ES Cell Medium, ReproStem (all by ReproCELL, Inc.), ProculAD (Rohto Pharmaceutical Co., Ltd.) sMSCBM-CD, MSCGM-CD (by Lonza Co.), EX-CELL302 medium (SAFC Corp.) or EX-CELL-CD-CHO (SAFC Corp.), ReproMed^{™} iPSC Medium (ReproCELL, Inc.), and mixtures of the aforementioned media.

If necessary, the medium may contain other medium additives, such as substances including one or more serum substitutes such as ROCK inhibitor, Knockout Serum Replacement (KSR), N2 supplement (Invitrogen), B27 supplement (Invitrogen), albumin, transferrin, apotransferrin, fatty acids, insulin, collagen precursors, trace elements, 2-mercaptoethanol and 3'-thiolglycerol, and it may also contain one or more substances such as lipids, amino acids, L-glutamine, Glutamax (Invitrogen), non-essential amino acids, vitamins, growth factors, low molecular compounds, antibiotics, antioxidants, pyruvic acid, buffering agents, inorganic salts, selenic acid, progesterone and putrescine. According to one aspect, the maintenance medium for primed pluripotent stem cells may be basal medium further containing bFGF and 20 essential amino acids, 10 vitamins (L-ascorbic acid, cobalamin, biotin, folic acid, I-inositol, niacinamide, D-calcium pantothenate, pyridoxine hydrochloride, riboflavin and thiamine hydrochloride), and 5 minerals (cupric sulfate, ferric sulfate, ferric nitrate, zinc sulfate and sodium selenite) (Scientific Reports volume 4, Article number: 3594 (2014)).

The culturing in the production method of the present invention may be by suspension culture or adhesion culture, but adhesion culture is preferred. As used herein, "suspension culture" is culturing carried out under conditions that maintain the state in which the cells or cell aggregates are suspended in the culture solution, or in other words, conditions such that no rigid cell-substratum junction is formed between the cells or cell aggregates and the culture vessel. Also, as used herein, "adhesion culture" is culturing under conditions such that a rigid cell-substratum junction is formed between the cells or cell aggregates and the culturing instrument.

The culturing vessel used for adhesion culture may be one where the surface of the culturing vessel is artificially treated to improve adhesion with cells (for example, coating treatment with extracellular matrix components such as basal membrane preparation, fibronectin or laminin or its fragments, entactin, collagen, gelatin, Synthemax or vitronectin, or a polymer such as polylysine or polyornithine, or surface processing by positive charge treatment). A culturing vessel coated with laminin or its fragments is preferred.

Laminin or its fragments to be used for the present invention include laminin-111 or its fragment containing the E8 domain, laminin-211 or its fragment containing the E8 domain (e.g. iMatrix-211), laminin-121 or its fragment containing the E8 domain, laminin-221 or its fragment containing the E8 domain, laminin-332 or its fragment containing the E8 domain, laminin-3A11 or its fragment containing the E8 domain, laminin-411 or its fragment containing the E8 domain (e.g. iMatrix-411), laminin-421 or its fragment containing the E8 domain, laminin-511 or its fragment containing the E8 domain (e.g. iMatrix-511, iMatrix-511 silk), laminin-521 or its fragment containing the E8 domain, laminin-213 or its fragment containing the E8 domain, laminin-423 or its fragment containing the E8 domain, laminin-523 or its fragment containing the E8 domain, laminin-212/222 or its fragment containing the E8 domain, and laminin-522 or its fragment containing the E8 domain.

The culture vessel used for suspension culture is not particularly limited so long as it allows "suspension culturing", and it may be selected as appropriate by a person skilled in the art. Examples of such culture vessels include a flask, tissue culture flask, dish, petri dish, tissue culture dish, multidish, microplate, microwell plate, micropore, multiplate, multiwell plate, chamber slide, petri dish, tube, tray, culture bag or roller bottle. A bioreactor is another example of a container for suspension culture. These culture vessels are preferably non-adhesive to cells to allow suspension culture. The non-cell-adhesive culture vessel used may also be one where the surface of the culture vessel is not artificially treated to improve adhesion with cells (for example, coating treatment with extracellular matrix).

The culturing temperature is not particularly limited but may be about 30 to 40°C and preferably about 37°C, with the culturing being carried out in a CO₂-containing air atmosphere with a CO₂ concentration of preferably about 2 to 5%.

The cell culture density is not particularly limited so long as the cells can proliferate. It will usually be 1.0 × 10² to 1.0 × 10⁷ cell/cm², preferably 1.0 × 10³ to 1.0 × 10⁶ cell/cm² and more preferably 1.0 × 10⁴ to 1.0 × 10⁵ cell/cm².

The production method of the present invention may further comprise a step of collecting the primed pluripotent stem cells obtained in step (2). Such a step, for example, may be carried out by detaching the cells from the culture vessel with a cell scraper after treatment with a cell dissociation enzyme (such as trypsin or a trypsin substitute (for example, TrypLE^{™} Select)), and collecting the detached cells using a pipette into a container such as a tube. The cells collected in the container may be passaged in a separate culture vessel, induced to differentiate, or cryopreserved using a cell cryopreservation solution (such as STEM-CELLBANKER). The cells collected in the container may also be counted using a cell counter, labeled with an antibody for a surface marker on primed pluripotent stem cells (such as CD24 or CD90), or purified by flow cytometry, mass cytometry or magnetic cell separation.

### 2. Method for increasing differentiation potency of pluripotent stem cells

As mentioned above, naive-inducing treatment of primed pluripotent stem cells can increase the differentiation potency of the pluripotent stem cells. Another aspect of the present invention, therefore, provides a method for increasing the differentiation potency of pluripotent stem cells, comprising (1) a step of inducing naive pluripotent stem cells from primed pluripotent stem cells, and (2) a step of inducing primed pluripotent stem cells from the naive pluripotent stem cells induced in step (1) (hereinafter sometimes referred to as "differentiation potency increasing method of the present invention").

Step (1) of the differentiation potency increasing method of the present invention may also comprise a step of sorting CD320-positive cells from the cell population. The differentiation potency increasing method of the present invention may comprise a step of collecting the pluripotent stem cells cultured in step (2). The culturing in the differentiation potency increasing method of the present invention may be culturing under feeder-free conditions and/or under xeno-free conditions for all or part of the culturing period. From the viewpoint of use in the clinic, the culturing in the differentiation potency increasing method of the present invention is preferably carried out under feeder-free and xeno-free conditions during the entire period.

The specific examples and definitions, etc., in the differentiation potency increasing method of the present invention, for the culturing methods and conditions including the cells used, the culturing period and the type of medium used, and for each step, are all incorporated as described above under "1. Method for producing primed pluripotent stem cells".

### 3. Differentiation-inducing method and differentiated cells obtained by the method

As explained above, the pluripotent stem cells of the present invention have high differentiation potency and can therefore be suitably used for differentiation of a variety of cell types. In another aspect of the present invention, therefore, a method for producing cells or an organoid, comprising a step of inducing differentiation of pluripotent stem cells of the present invention (hereinafter sometimes referred to as "differentiation-inducing method of the present invention"), is provided.

The cells obtainable by the differentiation-inducing method of the present invention may be undifferentiated cells such as stem cells or precursor cells, or the cells may be terminally differentiated cells. The term "differentiated cells" will also be used herein to encompass both undifferentiated cells and terminally differentiated cells. The cells may also be germ cells. As used herein, "undifferentiated cells" refers to cells that have not reached terminal differentiation in their cell lineage, with examples of undifferentiated cells including stem cells other than pluripotent stem cells, and precursor cells. Examples of stem cells and precursor cells include epiblast-like cells, primordial germ cells (also referred to as "primordial germ cell-like cells"), germ stem cells, ectodermal lineage cells such as neural crest cells, neural stem cells, neural progenitor cells, glial progenitor cells, retinal stem cells, corneal stem cells, keratinocyte epidermal stem cells, melanocyte stem cells and mammary stem cells, mesodermal lineage cells such as hematopoietic progenitor cells, myeloid stem cells, lymphoid stem cells, B cell progenitors, T cell progenitors, mesenchymal stem cells, cardiac stem cells, cardiac progenitor cells, vascular endothelial precursor cells, pericytes, platelet progenitor cells (e.g.: megakaryocyte progenitor cells, megakaryoblasts, promegakaryocytes and mature megakaryocytes), skeletal muscle stem cells, adipose stem cells, renal progenitor cells, and endodermal lineage cells such as hepatic stem cells, liver progenitor cells (e.g.: hepatoblasts, hepatic progenitor cells, hepatic stellate progenitor cells and hepatic stem cell precursors), intestinal stem cells and airway stem cells.

As used herein, the term "terminally differentiated cells" refers to cells that have reached terminal differentiation in their cell lineage, and examples of terminally differentiated cells include, but are not particularly limited to, osteoblasts, chondrocytes, adipocytes, hepatocytes, liver mesothelial cells, biliary epithelial cells, hepatic stellate cells, liver sinusoidal endothelial cells, Kupffer cells, pit cells, vascular endothelial cells, blood cells (e.g.: erythrocytes, platelets, leukocytes, mast cells and dendritic cells), pancreatic ductal epithelial cells, pancreatic ductal cells, centroacinar cells, acinar cells, islet of Langerhans cells, myocardial cells, fibroblasts, smooth muscle cells, type I alveolar epithelial cells, type II alveolar epithelial cells, clara cells, ciliated epithelium cells, basal cells, caliciform cells, neuroendocrine cells, Kulchitsky cells, tubular epithelial cells, urothelial cells, columnar epithelial cells, glomerular epithelial cells, glomerular endothelial cells, octopus cells, mesangial cells, neurons, and glial cells (e.g.: astrocytes, microglia, oligodendrocytes, ependymal cells and Schwann cells). Leukocytes include lymphocytes (e.g.: B cells, T cells and NK cells), granulocytes (e.g.: neutrophils, eosinophils and basophils), monocytes.

According to one embodiment of the present invention, the cells or organoid obtainable by the differentiation-inducing method of the present invention (the desired cells or organoid), are neural crest cells, neural progenitor cells, neurons, hematopoietic progenitor cells, platelets, T cells, epiblast-like cells, primordial germ cells or myocardial cells.

The term "organoid" as used herein means a construct formed by aggregation of cells, typically having a structure and function similar to an organ in the body. Examples of organoids to be obtainable by the differentiation-inducing method of the present invention include nerve organoids (e.g.: cerebral cortex organoid, cerebellum organoid, spinal cord organoid, mesencephalon organoid, choroid plexus organoid, hippocampus organoid, hypothalamus organoid, anterior pituitary gland organoid, cerebral basal ganglia organoid or retina organoid), lung organoid, liver organoid, airway epithelium organoid, intestinal organoid, pancreas organoid, kidney organoid, airway organoid, stomach organoid, thyroid gland organoid, thymus organoid, testes organoid, esophagus organoid, skin organoid, oviduct organoid, ovary organoid, salivary gland organoid, ophthalmic vesicle organoid, cupula optica organoid, bladder organoid, prostate organoid, cartilage organoid, heart organoid, bone tissue organoid, muscular tissue organoid and cancer organoids, and particularly nerve organoids such as cerebral cortex organoid, according to one embodiment.

That a given structure is an organoid can be confirmed, for example, by confirming the presence or absence of a layered structure formation using a microscope, or examining expression of marker proteins. For cerebral cortex organoid, specifically, a dome-shaped nerve epithelium is observed with expression of Foxg1 throughout, the nerve epithelium forming a layered structure. In this structure there is typically observed a layer of Pax6 and Sox2-positive neural progenitor cells corresponding to the ventricular zone, on the inner side of the epithelium, and a first layer composed of Reelin/Carletinin-positive Cajal Retzius cells and a Ctip2/Tbr1-positive deep layer, on the outer side. The markers are HHEX, SOX2, HNF4A, AFP and ALB for a liver organoid, the markers are PDX1, SOX17 and SOX9 for a pancreas organoid, the markers are CDX2 and SOX9 for an organoid that differentiates to intestinal tract, and the markers are Pax2 and Six2 for a kidney organoid.

The method of inducing differentiation to obtain the desired cells or organoid may be the method described in the following Examples, or a publicly known method. For example, inducing differentiation from pluripotent stem cells to neural crest cells may be carried out by the method described in Fukuta M. et al., PLoS One, 2014, 9(12): e112291 or Kamiya D, et al., NPJ Regen Med., 2022 Sep 15; 7(1):47. Specifically, pluripotent stem cells may be seeded on a culture vessel and adhesion cultured, and then caused to differentiate into neural crest cells by adhesion culture in medium containing a TGFβ inhibitor and GSK3β inhibitor.

From neural crest cells it is also possible to produce cells such as mesenchymal stem cells, neural progenitor cells, neurons, glial cells, osteocytes, chondrocytes, corneal cells and melanocytes. For example, inducing differentiation to these cell types may be carried out based on the method described in Fukuta M. et al., PLoS One, 2014, 9(12): e112291, Horikiri T. et al., PLoS One, 2017, 12(1): e0170342, or Kamiya D, et al., NPJ Regen Med., 2022 Sep 15; 7(1):47. Specifically, by seeding neural crest cells on a coated plate with fibronectin, exchanging the medium with DMEM/F12 supplemented with N-2 Supplement, BDNF, GDNF, NT-3 and NGF, and culturing the cells for about 14 days at 37°C, 5% CO₂, neural progenitor cells and neurons may be obtained. Alternatively, by seeding neural crest cells on a plate and culturing for 1 day in CDM medium containing SB431542 and CHIR99021, and then exchanging the medium with Neurobasal medium supplemented with B-27 Supplement, N-2 Supplement, L-glutamine, Penicillin/Streptomycin, BDNF, GDNF, NT-3 and NGF and cultured for about 35 days at 37°C, 5% CO₂, neural progenitor cells and neurons may be obtained.

The following method may be used as an example of inducing differentiation to mesenchymal stromal cells. Neural crest cells are seeded in a culture vessel and cultured for 1 day in CDM medium containing SB431542 and CHIR99021. After 1 day, the medium is exchanged with αMEM containing FBS. Mesenchymal stromal cells can be obtained after about 14 days from the start of inducing differentiation.

The method for differentiating pluripotent stem cells to T cells may be a method comprising, for example, (1) a step of causing differentiation of pluripotent stem cells to hematopoietic progenitor cells, and (2) a step of causing differentiation of the hematopoietic progenitor cells to T cells. For example, step (1) may be a step of culturing pluripotent stem cells in induction medium to hematopoietic progenitor cells, as described in WO2013/075222, WO2016/076415, Liu S.et al., Cytotherapy, 17 (2015); 344-358, etc. Step (2) may be (2-1) a step of inducing CD4, CD8 double-positive T cells from hematopoietic progenitor cells, and (2-2) a step of inducing CD8-positive T cells from CD4, CD8 double-positive T cells, as described in WO2016/076415, etc.

The step of inducing differentiation of pluripotent stem cells to platelets may be a method comprising, for example, (1) a step of causing differentiation of pluripotent stem cells to hematopoietic progenitor cells, and (2) a step of causing differentiation of the hematopoietic progenitor cells to platelets. For example, step (2) may be a step of culturing hematopoietic progenitor cells for about 7 to 15 days in medium containing TPO and/or SCF, as described in WO2012/157586, US2014/127815, Nakamura, Eto, et al. Cell Stem Cell 14, 535-548 (2014), etc. This step can produce a cell population which includes megakaryocytes and platelets.

A step of inducing differentiation of pluripotent stem cells to primordial germ cells, for example, may be a method comprising (1) a step of culturing pluripotent stem cells for about 40 hours to 60 hours in culture solution containing activin A and GSK3β inhibitor to cause differentiation to epiblast-like cells, and (2) a step of culturing the epiblast-like cells for about 4 to 8 days in culture solution containing BMP to cause differentiation to primordial germ cells (primordial germ cell-like cells), as described in WO2017/002888, etc.

Examples of inducing differentiation from pluripotent stem cells to myocardial cells include methods described in WO2015/141827, Laflamme MA and Murry CE, Nature. 473(7347):326-35 (2011), etc. Other examples are a method of producing myocardial cells by forming an embryo body by suspension culture of induced pluripotent stem cells, a method of producing myocardial cells in the presence of a substance that inhibits BMP signaling (WO2005/033298), a method of producing myocardial cells with addition of Activin A and BMP in that order (WO2007/002136), and a method of producing myocardial cells in the presence of a substance that promotes activation of the canonical Wnt signaling pathway (WO2007/126077), etc.

A nerve organoid can be produced by culturing a cell aggregate containing neural progenitor cells induced by the SFEBq method (typically formed around day 18 from the start of inducing differentiation), in differentiation-inducing medium for the desired neurons, as described in, for example, Sasai Y. et al., Cell Stem Cell. 12:520-530 (2013), Sakaguchi H. et al., Nat Commun. 6:8896 (2015), etc. Continued culturing of the cell aggregate in such neuron differentiation-inducing medium under suspension culture conditions can induce formation of a nerve organoid by self-assembly. The SFEBq method typically includes a step in which primed pluripotent stem cells dispersed with an enzyme are suspension cultured in medium containing a Wnt signal inhibitor, TGF-β inhibitor and ROCK inhibitor on the first day of inducing differentiation (Day 0), and cultured from Day 1 onward in medium containing a Wnt signal inhibitor and TGF-β inhibitor.

Examples of Wnt signal inhibitors to be used in the SFEBq method include DKK1 protein, sclerostin, IWR-1e, IWP-2, IWP-3, IWP-4, IWP-L6, C59, ICG-001, FH535, WIKI4, KYO2111, PNU-74654 and XAV939, as well as their derivatives. Examples of TGF-β inhibitors include Lefty-1, SB431542, SB202190, SB505124, NPC30345, SD093, SD908, SD208, LY2109761, LY364947, LY580276 and A-83-01, as well as their derivatives. ROCK inhibitors include the same ones mentioned above.

Examples of neuron differentiation-inducing media include, but are not limited to, N2 medium for inducing cerebral cortex organoid (medium prepared with addition of N2 supplement to DMEM/F12 medium), medium containing CHIR99021 and BMP4 for inducing choroid plexus organoid, and medium containing SAG, BMP4 and KSR for inducing pituitary organoid. The neuron differentiation-inducing medium may be changed as appropriate during induction of the organoid.

Different types of cells may also be used for production of an organoid. For a liver organoid, for example, hepatic progenitor cells (organ cells), mesenchymal stem cells and vascular endothelial cells may be induced from pluripotent stem cells as described in WO2013/047639, etc., and the mixture may be suspension cultured to produce a liver organoid.

The differentiation-inducing method of the present invention may be culturing under feeder-free conditions and/or under xeno-free conditions, for all or part of the culturing period. From the viewpoint of use in the clinic, the culturing in the differentiation-inducing method of the present invention is preferably carried out under feeder-free and xeno-free conditions during the entire period.

In another aspect of the present invention, cells or an organoid obtained by the differentiation-inducing method of the present invention (hereinafter also referred to as "differentiated cells or organoid of the present invention") are provided.

The differentiation-inducing method of the present invention may comprise a step of collecting the obtained desired cells or organoid. The collected cells may be cryopreserved using a cell cryopreservation solution. The cells collected in the container may also be counted using a cell counter, labeled with an antibody for a cell surface marker, and purified by flow cytometry, mass cytometry or magnetic cell separation.

The specific examples and definitions, etc., in the differentiation-inducing method of the present invention, for the culturing methods and conditions including the cells used, the culturing period and the type of medium used, and for the collecting step, are all incorporated as described above under "1. Method for producing primed pluripotent stem cells".

### 4. Use of differentiated cells or organoid

Since the differentiated cells or organoid of the present invention can be suitably used for immunotherapy or regenerative medicine, in another aspect of the present invention, a medicament comprising the differentiated cells or organoid of the present invention (hereinafter also referred to as "medicament of the present invention"), is provided. For example, the medicament of the present invention may be provided in the form of an agent, such as an immunotherapy agent or a cell grafting agent. The present invention also encompasses a method for treating disease in which an effective amount of the differentiated cells or organoid of the present invention is administered or grafted into a primate to be treated. Specific examples of primates include those mentioned above under "1. Method for producing primed pluripotent stem cells", but humans are preferred.

The differentiated cells or organoid of the present invention may be used by administration or grafting into the body of a subject in need of the same. Grafting is preferably carried out in a region of the body where the cells can be anchored at a fixed location, such as under the skin, in the peritoneal cavity, in the peritoneal epithelium, in the omentum, or in adipose tissue or muscle tissue, or in the subserosal layer of an organ such as the pancreas or kidneys. Grafting is preferably subcutaneous for a lower degree of invasiveness. The grafted cells may be administered in a therapeutically effective amount, which may be adjusted according to the age, body weight and graft site size of the subject to receive the graft, and on the severity of symptoms, and may be about 10 × 10⁴ cells to 10 × 10¹¹ cells, for example, without being limitative.

When differentiated cells or an organoid of the present invention is to be used as a medicament, it is preferred to use cells or an organoid derived from iPS cells established from somatic cells having the same or essentially the same HLA genotype as the recipient individual to be grafted, from the viewpoint of avoiding rejection reaction. Here, "essentially the same" means that the HLA genotype matches to a degree that allows immunoreaction against the grafted cells to be suppressed with immunosuppressive agents, and examples are somatic cells having an HLA type that matches at the 3 gene loci HLA-A, HLA-B and HLA-DR, or 4 gene loci further including HLA-C. When a sufficient number of cells cannot be obtained due to age or constitution, they may be grafted in a state embedded in capsules or a porous container of polyethylene glycol or silicone, to avoid rejection reaction.

The differentiated cells or organoid of the present invention are produced as a parenteral dosage form such as an injection, suspending agent or intravenous drip, for example, by combining with a pharmaceutically acceptable carrier according to conventional means. Therefore, in one aspect of the present invention, a method for producing an immunotherapy agent or cell graft treatment agent, comprising formulation of differentiated cells or an organoid of the present invention, is provided. The method may also comprise a step of preparing the differentiated cells or organoid of the present invention. The method may also comprise a step of storing the differentiated cells or organoid of the present invention.

Examples of pharmaceutically acceptable carriers that may be included in parenteral dosage forms include aqueous solutions for injection, such as physiological saline, or isotonic solutions containing glucose or other adjuvants (such as D-sorbitol, D-mannitol and sodium chloride). The cells of the present invention may be formulated with, for example, a buffering agent (such as phosphate buffer or sodium acetate buffer), a soothing agent (such as benzalkonium chloride or procaine hydrochloride), a stabilizer (such as human serum albumin or polyethylene glycol), a preservative or an antioxidant, etc.

The immunotherapy agent or cell graft treatment agent of the present invention may also be provided in a cryopreserved state under normal conditions used for cryopreservation of cells, and thawed at the time of use. In this case, it may further comprise serum or a serum substitute, or an organic solvent (such as DMSO), etc. In this case, the concentration for serum or a serum substitute is not particularly limited but may be about 1 to about 30% (v/v), and preferably about 5 to about 20% (v/v). The concentration for an organic solvent is not particularly limited but may be about 0 to about 50% (v/v), and preferably about 5 to about 20% (v/v).

The differentiated cells or organoid of the present invention may also be used in a method of screening of candidate medicaments as medicaments useful for treatment or prevention of a disease.

The present invention will now be described in greater detail by Examples, with the understanding that the present invention is not in any way limited to these Examples.

### EXAMPLES

### Example 1: Induction of naive pluripotent stem cells (reset cells) from primed pluripotent stem cells

### 1. Induction of reset cells

Reset cells were induced by a previously reported method (NPLs 1 to 3) from human primed pluripotent stem cell lines (derived from H9ES cells, H1ES cells, AdiPS, 1383iPS cells, etc.) (culture scale: 6 well plate). Specifically, the reset cells were induced by the following method.

A human primed pluripotent stem cell (PSC) line (H9ES (Embryonic Stem) cells, H1ES cells and AdiPS cells) was maintained on γ-ray-irradiated MEFs under conventional conditions ("F12/KSR") (Dulbecco's modified Eagle medium [DMEM/F12; Nacalai Tesque, Inc., Cat. 08460-95], 20%[v/v] KSR [Thermo Fisher Scientific, Cat. 10828028], nonessential amino acids [NEAA; Thermo Fisher Scientific, Cat. 11140-050], 4 ng/ml recombinant human bFGF [bFGF; Oriental Yeast Co., Ltd., Cat. NIB 47079,000], 0.1 mM 2-mercaptoethanol [Sigma-Aldrich, Cat. M3148]). The cells were detached with a small clamp every 5 to 7 days using Dissociation Buffer (DB; 0.025% Trypsin [Thermo Fisher Scientific, Cat. 15090-046], 1 mg/ml Collagenase IV [Thermo Fisher Scientific, Cat. 17104-019], 20% KSR, 1 mM CaCl₂), and passaged.

A human naive pluripotent stem cell line (derived from H9ES cells, H1ES cells or AdiPS cells) was maintained on MEFs using t2iLGo (Ndiff227 [Takara Bio, Cat. Y40002], 1 µM PD0325901 [PD03; Tocris, Cat. 4192], 1 µM CHIR99021 [CH; Sigma-Aldrich, Cat. SML1046], 10 ng/ml Recombinant human LIF [hLIF; Peprotech, Cat. 300-05], 3 µM Go6983 [Go; Tocris, Cat. 2285]). The cells were detached using Accutase (Sigma-Aldrich, Cat. A6964) every 3 to 5 days and passaged.

The naive H9PSCs were established by a method using an HDAC inhibitor (Guo, G. et al. (2017). Development 144(15): 2748-2763.). The establishment method using an HDAC inhibitor is also referred to "chemical resetting". During establishment, EOS plasmid was introduced into the primed H9PSCs by electroporation (primed H9 EOS). A gene serving as a drug resistance marker (puromycin resistance) was incorporated into the plasmid, and after introduction into the cells, Puromycin Dihydrochloride (Thermo Fisher Scientific, Cat. A1113802) was used for drug selection for selection of cells having the plasmid introduced. The primed H9 EOS were detached with trypsin/EDTA (Nacalai Tesque, Inc., Cat. 32777-15) into single cells, and 1 × 10⁵/cm² cells were seeded on MEFs in F12/KSR medium supplemented with 10 µM Y-27632 (Wako, Cat. 034-24024). From the following day, culturing was carried out for 48 hours in cRM1(Ndiff, 1 µM PD03, 10 ng/ml hLIF, 1 mM valproic acid sodium salt [VPA; Sigma-Aldrich, Cat. P4543]), after which the cells were maintained with cRM2 (Ndiff, 1 µM PD03, 10 ng/ml hLIF, 2 µM Go, 2 µM XAV939 [Sigma-Aldrich, Cat. X3004]). Around the 3^{rd} to 5^{th} passages (approximately day 20), most of the cells form dome-shaped colonies. At this point, culturing was switched to t2iLGo medium to establish the cells.

In some experiments, 5iLFA conditions have been used to establish naive H9 Theunissen et al., Cell Stem Cell. 2016 Oct 6; 19(4):502-515). The primed H9 EOS were detached with trypsin/EDTA into single cells, and 1 × 10⁵/cm² cells were seeded on MEFs in F12/KSR medium supplemented with 10 µM Y-27632. From the following day, the medium was changed to 5iLFA medium (Ndiff, 1 µM PD03, 1 µM CH, 1 µM WH-4-023 [A Chemtek H620061], 0.5 µM SB590885 [R and D 2650], 10 µM Y-27632, 10 ng/ml hLIF, 20 ng/ml Activin A [R&D, Cat. 388-AC], 8 ng/ml bFGF) and culturing was continued. When most of the cells had formed domeshapd colonies (3^{rd} to 5^{th} passages (approximately day 20), the cells were maintained by switching to t2iLGo to establish the cells.

Induction of naive pluripotent stem cells under freeder-free conditions can be carried out by the following method. The cell morphology of primed iPS or ES cells that have been maintenance cultured 2 days before start of induction (Day -2) is confirmed (the morphology of the primed cells shown in Fig. 1). A coating plate is prepared with iMatrix511 (0.5 µg/cm²). The primed iPS or ES cells are then incubated for 1 hour or longer after addition of Y27632 at a final concentration of 10 µM. The cells are detached with Accutase and collected in 1 mL of wash buffer dispensed in a 15 mL tube. After centrifugation, the cells are resuspended in AK02N + Y27632 (final concentration: 10 µM) and counted. After then dispensing AK02N + Y27632 (final concentration: 10 µM) at 1.5 mL/well into the coated plate, 1 x 10⁵ cells/well are seeded. Culturing is initiated at 37°C, 5% O₂, 5% CO₂. Medium exchange is not carried out on the day before starting induction (Day -1). At the start of inducing differentiation (Day 0, 12:00 am), the cultured cells are washed once with PBS and the medium is exchanged with cRM-1 + Y27632 (final concentration: 10 µM) (1.5 mL/well). Medium exchange is not carried out on Day 1 (after 24 hours). The medium is exchanged with cRM-1 + Y27632 (final concentration: 10 µM) (1.5 mL/well) on Day 2 (after 48 hours). On Day 3 (after 72 hours), the cultured cells are washed once with PBS and the medium is exchanged with cRM-2 + Y27632 (final concentration: 10 µM) (1.5 mL/well). Medium exchange is carried out with cRM-2 + Y27632(final concentration: 10 µM) (1.5 mL/well) every other day from Day 3 until the 1st passage. Appearance of dome-shaped naive pluripotent stem cell colonies is confirmed from Day 9 to Day 12 (for example, colonies as indicated on day 9 in Fig. 1), and the 1^{st} passage is carried out.

For the 1^{st} passage, a coating plate is prepared with iMatrix511 (0.13 µg/cm²). The cultured cells are washed once with PBS and detached with Accutase/EDTA, and then collected in wash buffer dispensed in a 15 mL tube. After centrifugation, the cells are suspended in cRM-2 medium and counted with Trypan blue. CRM-2 + Y27632 (final concentration: 10 µM) is dispensed into a prepared coated plate at 1.5 mL/well, and the cells are seeded at 1 x 10⁵ cells/well. Culturing is initiated at 37°C, 5% O₂, 5% CO₂. Medium exchange is not carried out on Day 1. Medium exchange is carried out each day from Day 2 to Day 5 with cRM-2 at 1.5 mL/well.

From the 2^{nd} passage onward, iMatrix511 is added for culturing without pre-coating of the cell culturing vessel. The cultured cells are washed with PBS and detached with TrypLE + CDS, and then collected in wash buffer dispensed in a 15 mL tube. After centrifugation, the cells are resuspended in cRM-2 medium and counted with Trypan blue. After dispensing cRM-2 + Y27632 (final concentration: 10 µM) into the cell culturing vessel at 1.5 mL/well, the cells are seeded at 1 x 10⁵ cells/well. The cells are quickly and evenly seeded after addition of iMatrix511 at 3.75 µL/well (0.19 µg/cm²). Culturing is initiated at 37°C, 5% O₂, 5% CO₂. Medium exchange is not carried out on Day 1. Medium exchange is carried out each day from Day 2 to Day 5 with cRM-2 at 1.5 mL/well. Passage is carried out every 5 days after Day 4 from the 2^{nd} passage onward. Small dome-shaped naive pluripotent stem cells of about 50 µm to 100 µm are observed (cells as indicated by Day 14, Day 19 and Day 24 in Fig. 1, for example), and after at least 2-3 passages, relatively homogeneous, small dome-shaped naive pluripotent stem cells are observed (for example, cells like the reset cells in Figure 1).

### 2. Verification of change in surface antigens

Naive pluripotent stem cells induced from a human primed pluripotent stem cell line (derived from H9ES cells, H1ES cells, or AdiPS, 1383 iPS cells, etc.) were analyzed with Flow Cytometers (FCM), by the following method.

The naive iPS cells were washed twice with PBS during culturing, and TrypLE + CDS was added to detach the cells. After centrifugation, the cells were resuspended in Hanks buffer + Y27632(final concentration: 10 µM), a cell count necessary for FCM analysis was separated off into a 1.5 mL tube, and blocking was carried out for 20 minutes on ice with Hanks buffer + Y27632 (final concentration: 10 µM). After centrifugation the supernatant was removed and the cells were suspended in Hanks buffer + Y27632 (final concentration: 10 µM) containing antibodies necessary for analysis (CD24, CD57, SUSD2, CD75). After conducting antigen-antibody reaction for 20 minutes on ice and washing the cells, DAPI was added to Hanks buffer + Y27632 (final concentration: 10 µM) for suspension of the cells, and FCM analysis was carried out.

The results showed that for the previously reported marker genes, the primed-type markers CD24 and CD57 were lost by day 9 of induction, while the naive-type markers SUSD2 and CD75 were expressed (Day 9 in Fig. 2).

### 3. Comprehensive gene expression analysis

Comprehensive gene expression analysis (RNA sequencing, RNA-seq) and principal component analysis (PCA) were carried out next. Specifically, RNA was extracted from the cells, and an Illumina kit was used to construct a library from 200 ng of total RNA. A NextSeq500 was used for sequencing. The expression level was standardized and analyzed using edgeR.

PCA showed a difference in gene expression pattern between the cells on day 9 of induction (D9) and the reset cells (Fig. 3). Upon single cell RNA sequencing (scRNA-seq), it was found that the reset cells were cells of cluster 6 (CL6), and that a few cells corresponding to CL6 began to appear around day 14, with most of the cells being reset cells by day 24. The results indicated more time required than previously reported (Fig. 4).

### 4. Verification of differentiation potency to primitive endoderm and miRNA expression

It is known that reset cells are able to differentiate into extraembryonic cells, i.e. the primitive endoderm (yolk sac) and trophectoderm (placental cells), but primed iPS cells cannot. The differentiation potency to primitive endoderm cells was therefore examined. With induction using PDGF-AA, IL-6, TGFβ inhibitor, Wnt signal inhibitor and retinoic acid in addition to BMP4 (Bone morphogenetic protein) and FGF4 (Fibroblast growth factor 4), it was found that differentiation from D15 to primitive endoderm (a type of extraembryonic cell) takes place (Fig. 5), similar to the results of scRNA-seq. Upon subsequent searching for marker genes expressed by reset cells using qPCR expression analysis, the surface antigen CD320 was successfully identified (Fig. 6). CD320 is in fact known to have high expression in the human preimplantation epiblast. It was shown that CD320 appears around day 14 during naive induction. When CD320-positive cells were purified and collected, the CD320-high positive cells exhibited the gene expression and morphology of reset cells (Fig. 7). The CD320-low positive cells, however, had a differentiated morphology, with low expression of genes associated with reset cells, with low differentiation potency to primitive endoderm as well (Fig. 7).

The qPCR expression analysis was carried out as follows. The total RNA was extracted with an RNeasy Kit (Qiagen, Cat. 74106), and cDNA was synthesized from 1000 ng of RNA using SuperScript IV (Thermo Fisher Scientific, Cat. 18090050) and oligo-dT primer. TaqMan Probe or powerUP Sybr Green Master Mix (Thermo Fisher Scientific) was used for real-time PCR. Analysis after the real-time RT-PCR reaction was carried out using QuantStudio Design & Analysis Software v1.4.1.

As a result of examining expression of miRNA by activation using miRNA switches, miR-520c-3p and miR-520h were found to match reset cell differentiation and increase activity (Fig. 8). Human primed H9 cells containing introduced EOS (Early Transposon promoter and Oct-4 (Pou5f1) and Sox2 enhancers) - GFP reporter gene were used for this analysis. The EOS-GFP reporter gene has a binding region for OCT3/4 and SOX2 present in the Oct-4 distal enhancer, and in the naive state this is activated, resulting in expression of GFP. That is, GFP is not expressed by somatic cells or primed pluripotent stem cells, but is expressed by naive pluripotent stem cells. Since EOS-GFP positive cells began expression on day 14, this demonstrated expression in reset cells (Fig. 9). The proportion of EOS-GFP positive cells already reached about the same level as the control (human naive type H9 cells) on day 14 (Fig. 9).

It was thus demonstrated that reset cells can in fact be obtained from day 14 onward during induction of the naive cells from primed cells, and a marker (CD320) was successfully identified that can be used for reset cells.

The reagents used in the Examples are listed in Table 2. The number of cells seeded in the Examples was between about 0.5 x 10⁵ and 5 x 10⁵ cells/well, depending on the cell line. The types of reagents and reagent manufacturer may be different so long as they have similar performance as the reagents listed in Table 2. The culture vessel coating agent used may also be a common one that allows culturing of primed iPS cells such as laminin, vitronectin, fibronectin, Matrigel or Geltrex, instead of iMatrix511. The cells can also be cultured on MEFs.

**[Table 2]**

| Chemical Resetting Medium 1: cRM-1 | | |
|---|---|---|
| Takara Ndiff227 | Takara | 10ml |
| PD0325901 (stock: 10 mM) | TOCRIS #4192 | 1 µl (final: 1 µM) |
| Human LIF (stock: 10 µg/ml) | Peprotech #300-05-1mg | 10 µl (final: 10 ng/ml) |
| VPA (stock: 0.3 M) | SIGMA #P4543-10G | 33 µl (final: 1 mM) |

### Chemical Resetting Medium 2: cRM-2

| Takara Ndiff227 | Takara | 10 ml |
|---|---|---|
| PD0325901 (stock: 10 mM) | TOCRIS #4192 | 1 µl (final: 1 µM) |
| Human LIF (stock: 10 µg/ml) | Peprotech #300-05-1mg | 10 µl (final: 10 ng/ml) |
| Go6983 (stock: 5 mM) | TOCRIS #2285 | 4 µl (final: 2 µM) |
| XAV939 (stock: 20 mM) | Selleck S1180 | 1 µl (final: 2 µM) |

### Wash buffer

| | | |
|---|---|---|
| DMEM/F12 | Nacalai #11581-15 | 500 ml |
| 7.5% BSA | Wako #012-23881 | 6.76 ml (final: 0.1%) |

### TrypLE+CDS

| | |
|---|---|
| TrypLE^{™} Express Enzyme (1X), no phenol red | 5 mL |
| Cell dissociation solution (#S-004-C) | 5 mL |

### Accutase+EDTA

| | |
|---|---|
| Accutase | 5 mL |
| 5.0 µmol/l-EDTA solution | 5 mL |

### Example 2: Induction from naive pluripotent stem cells to primed pluripotent stem cells with high differentiation-inducing efficiency

Primed pluripotent stem cells (hereinafter also referred to as "reprimed pluripotent stem cells") were induced from human primed pluripotent stem cell lines (from H9ES cells, 585B1 iPS cells, 1231A3 iPS cells, 1383D6 iPS cells, 585B1 iPS cells, 201B7 iPS cells, 409B2 iPS cells or I14S04 iPS cells) by way of naive pluripotent stem cells (representative cell morphology shown in Fig. 10) (culturing scale: 6-well plate). Specifically, the reprimed pluripotent stem cells were induced by the following method.

Naive pluripotent stem cells obtained as single cells by a method of coating with iMatrix-511 (Matrixome Co.) to 0.5 µg/cm² and detaching the naive pluripotent stem cells (the same method as for detaching the cells described under the method of passage from 2^{nd} Passage onward in Example 1) were seeded into a 6-well plate at 1 x 10⁵/well, and cultured in StemFit AK02 medium (Ajinomoto Healthy Supply Co) + Y-27632 (final concentration: 10 µM), begun at 37°C, 5% O², 5% CO₂. 2 days after seeding, the medium was exchanged with medium lacking Y-27632 (final concentration: 10 µM). The medium was exchanged with StemFit (AK02) medium every day until the cells reached confluence. This method induced primed pluripotent stem cells from the naive pluripotent stem cells by about 8 days. Fig. 10 shows the morphology of the H9 and 585B1 iPS cells.

Analysis of the reprimed pluripotent stem cells with Flow Cytometers (FCM) was carried out by the following method. The naive iPS cells in the medium were washed twice with PBS. The cleaning fluid was removed and the cells were detached with Accutase, and then collected in wash buffer that had been dispensed in a 15 mL tube. After centrifugation, they were resuspended in 1 mL of Hanks buffer + Y27632 (final concentration: 10 µM), and the cells were counted with Trypan blue. The reprimed pluripotent stem cells in a cell count necessary for FCM analysis were dispensed in a 1.5 mL tube, and blocking was carried out for 20 to 30 minutes in 50 µL of Hanks buffer + Y27632 (final concentration: 10 µM) on ice. Antibodies (CD24, CD57, SUSD2, CD320) were then added to the Hanks buffer + Y27632 (final concentration: 10 µM) in amounts necessary for analysis, and antigen-antibody reaction was conducted for 20 to 30 minutes on ice. After washing the cells, the cells were suspended in Hanks buffer + Y27632 (final concentration: 10 µM) for suspension of the cells, DAPI was added while the cells were suspended, and FCM analysis was carried out.

Fig. 11 shows the transition in surface antigens when reprimed pluripotent stem cells were induced from primed pluripotent stem cells by way of the naive state. From day 9 onwards after starting induction of naive pluripotent stem cells to reprimed pluripotent stem cells by the method described above, the naive pluripotent stem cell markers SUSD2 and CD320 disappeared, and the primed pluripotent stem cell markers CD24 and CD90 were expressed (Fig. 11).

The reagents used in the Examples are listed in Table 3. The culture vessel coating agent used may also be a common one that allows culturing of primed pluripotent stem cells such as laminin, vitronectin, fibronectin, Matrigel or Geltrex, instead of iMatrix511. The cells can also be cultured on MEFs. The cell dissociation solution used in the Examples was a mixture of TrypLE (Thermo Fisher) and cell dissociation solution in equal amounts, but it may also be Accutase or TrypLE which are commonly used for primed pluripotent stem cells. The culture solution used for culturing of the primed iPS cells may be any culture solution that allows culturing of common primed pluripotent stem cells, such as StemFit AK02, or mTeSR1 or Essential 8 medium (Gibco). The seeded cell count may be 1 x 10⁴ to 1 x 10⁵/well so long as the cells do not reach confluence by about 4 days, although the cell proliferation rate will differ depending on the cell line.

**[Table 3]**

| TrypLE+CDS | |
|---|---|
| TrypLE^{™} Express Enzyme (1X), no phenol red | 5 mL |
| Cell dissociation solution (S-004-C) | 5 mL |

### Example 3: Verifying differentiation potency of reprimed pluripotent stem cells

The differentiation potency of primed pluripotent stem cells and reprimed pluripotent stem cells was evaluated. The results are summarized in Fig. 12.

### 1. Inducing neurons and myocardial cells

Differentiation potency to neurons and myocardial cells was verified. For neural induction, 100 nM LDN-193189 and 10 µM SB431542 were added to Ndif227 medium, and after 10 days of culturing, PAX6-FITC expression was quantified by flow cytometry. A PSC Cardiomyocyte Differentiation Kit (ThermoFisher, Product No. A2921201) was used for cardiac muscle induction. On day 10, the number of wells with pulsating myocardial tissue was counted on the 10th day.

The results are shown in Fig. 13. When the lines with low differentiation potency to neurons and myocardial cells in the primed state were converted to the re-primed state by the method of Example 2, the cells exhibited increased differentiation potency to neurons and myocardial cells (Fig. 13).

### 2. Inducing human primordial germ cell-like cells (hPGCLC)

The differentiation potency to PGCLC was verified. Human primed pluripotent stem cell lines (derived from H9ES cells, H1ES cells, 585B2iPS, 201B7iPS, 409B2iPS cells, etc.) were used. iPS cells transiently induced to the primed state from the transient naive type in Example 2 (i.e. reprimed iPS cells) were used. After suspending 2.5 to 3.5 × 10³ primed iPS cells in 150 µL of hPGCLC differentiation-inducing medium, the cells were seeded in 1 well of low-cell-biding V-bottom 96 well plates (MS-9096 V; Sumitomo Bakelite). The hPGCLC differentiation-inducing medium used was Glasgow's MEM (11710-035; Gibco) containing 15% [v/v] KSR (10828028; Gibco), 0.1 mM NEAA (11140-050; Gibco), 2 mM L-glutamine (25030-081; Gibco), 1 mM sodium pyruvate (11360-070; Gibco), 0.1 mM 2-mercaptoethanol (M3148; Sigma-Aldrich), 10 ng/mL LIF (300-05; Peprotech), 200 ng/ml BMP4 (314-BP; R&D systems), 100 ng/ml SCF (300-07; Peprotech), 50 ng/ml EGF (236-EG; R&D systems) and 10 µM Y-27632 (10005583; Cayman). Culturing was carried out for 5 days after seeding without medium exchange.

After culturing, the hPGCLC differentiation-inducing efficiency was evaluated using the following antibody-positive cells as indicator. hPGCLCAPC anti-human CD326 (EpCAM) Antibody (BioLegend, Cat#324208), Brilliant Violet 421^{™} anti-human/mouse CD49f (ITGA6) Antibody (BioLegend, Cat#313623).

The results are shown in Fig. 14. Cell lines that did not differentiate into hPGCLC or had low differentiation potency to hPGCLC in the primed state, when converted to the re-primed state by the method of Example 2, exhibited increased differentiation potency to hPGCLC (CD326+ CD49f+ cells).

### 3. Induction of neural crest cells

Differentiation potency to neural crest cells was verified. Primed cells and reprimed cells of the 1383D6 line were maintained in a culture dish coated with iMatrix (laminin-511 E8 fragment) and containing Stemfit AK03N xeno-free medium (chemically known and containing no animal components) (Sci Rep 4, 3594(2014)). Before starting induction to neural crest cells, the cell lines were cultured for 4 days until colonies formed. After 10 days from inducing neural crest cells in Stemfit Basic03 medium supplemented with 10 µM SB431542 and 1 µM CHIR99021 (CHIR), fluorescence activated cell sorting (FACS) was used to analyze the induction efficiency, using antibody for the neural crest cell surface marker CD271.

The results are shown in Fig. 15. A cell population induced to differentiate from reprimed pluripotent stem cells prepared by the method of Example 2 exhibited a higher proportion of cells with high expression of CD271 (CD271-high positive cells), as a neural crest cell marker, compared to the cell population induced to differentiate from primed pluripotent stem cells. Of the histograms shown in Fig. 15, the peaks observed in the range of about 10³ to 3 x 10⁴ cells, mainly seen in the primed state, are cells other than neural crest cells, and are referred to as "CD271-low positive cells" (CD271^{dim} cells). The peak observed in the range of about 10⁴ to 10⁵, seen with the re-primed cells, are neural crest cells, and are referred to as "CD271 high-positive cells" (CD271^{high} cells). While virtually no CD271 high-positive cells (neural crest cells) were produced from the primed pluripotent stem cells, a very large number of CD271 high-positive cells (neural crest cells) were produced from the reprimed pluripotent stem cells, thus indicating that differentiation potency to neural crest cells is drastically increased by conversion to the re-primed state.

### 4. Induction of blood cells

### 4-1. Induction of platelets

The differentiation potency to platelets was verified. Using the method described in WO2012/157586, US2014/127815, Nakamura, Eto, et al. Cell Stem Cell 14, 535-548 (2014), Sone, and Nakamura, Eto, et. al Stem Cell Reports, 16(12):2861-2870(2021), cells of a megakaryocyte line (imMKCL) derived from primed pluripotent stem cells and imMKCL derived from cells induced to naive pluripotent stem cells from primed pluripotent stem cells and then induced back to primed pluripotent stem cells (re-primed cells) were cultured for 6 days in the maturation culturing solution described in Ito, Nakamura, Eto et al. Cell, 174(3):636-648(2018), and the culture solution was collected. The collected culture solution was labeled with CD41a and CD42b antibody, and BD Trucount^{™} was used to count the CD41a and CD42b double-positive platelets by flow cytometry.

The results are shown in Fig. 16. When differentiation was induced from the reprimed iPS cells prepared by the method of Example 2, the differentiation potency to platelets was increased compared to inducing differentiation from primed iPS cells.

### 4-2. Induction of T cells

The differentiation potency to T cells was verified next. The differentiation-inducing protocol from iPS cells to CD8-positive T cells was as follows. First, an embryo body (EB) was prepared by adding WNT, BMP, bFGF and VEGF and SCF to the medium. After 2 weeks, the CD34-positive cells were collected and CD4 CD8 double-positive (DP) cells were induced for about 20 days using SCF, TPO, FLT3L and IL-7 (Iriguchi S et al., Nature Communications, 2021 Jan 18; 12(1):430.). CD8 single-positive (D8 SP) cells were then induced for about 1 week using OKT3, IL-7 and IL21, and the cells were maturated (Kawai Y et al., Mol Ther, 2021 Oct 6; 29(10):3027-3041). The cells were maintained using PHA, IL-7 and IL-15.

The results are shown in Figs. 17 to 19. It was shown that even with induction to T cells, the iPS cell line with low differentiation potency in the primed state exhibited increased differentiation potency to T cells by conversion to the re-primed state by the method of Example 2. The re-primed type could more effectively induce CD4+/8b+, CD5+/CD28+ and CD1+/CD28+ cells (Fig. 17). The differentiation potency to CD5+/CD28+ and CD8+ SP cells also increased (Fig. 18). Maintenance and expansion culturing of the CD8-positive cells was also possible (Fig. 19).

### 4-3. Induction of cerebral cortex organoid

It was shown that even when inducing differentiation of a cerebral cortex organoid, the iPS cell line with low differentiation potency in the primed state exhibited increased differentiation potency to cerebral cortex organoid by conversion to the re-primed state by the method of Example 2. Fig. 20 shows an overview diagram of the induction method. The culturing and evaluating methods are described in detail below.

### <Maintenance culture of iPS cells>

Culturing was carried out in StemFit AK02N medium in an iMatrix-coated 6-well plate. On the day before inducing differentiation (d-1), 5 µM of SB431542 was added to the AK02N-bFGF medium (bFGF-free AK02N medium), and culturing was initiated. On the first day of inducing differentiation (d0), enzyme-dispersed undifferentiated iPS cells were seeded in a 96-well plate at 9,000 cells/well. Culturing was carried out in DMEM/F12 medium supplemented with 20% KSR, 3 µM IWR-1e and 5 µM SB431542, exchanging the medium once every 3 days. On the first day of inducing differentiation (only), 50 µM of Y-27632 was added. On day 18 of inducing differentiation, the organoid was transferred to the 96-well plate to a 90-mm dish and cultured with DMEM/F12 supplemented with 1 × N2 supplement and Chemically Defined Lipid Concentrate (CDLC). Gyratory culture was carried out at 50 rpm thereafter, exchanging the medium once every 3 to 4 days. On day 35, the organoid was photographed with a stereomicroscope and the efficiency of organoid formation containing a rosette structure was calculated. The re-primed cells obtained by way of naive pluripotent stem cells had formed a well-formed FOXG1/CTIP2/PAX6/DAPI-positive cerebral cortex organoid (Fig. 21). The percentage of rosette-containing organoids was also increased (Fig. 22).

### Example 4: Verification of differentiation potency of primed pluripotent stem cells induced from naive type cells induced without transitioning through primed pluripotent stem cells

Human somatic cells (HDF cells and hADSC cells) were used. HDF cells (Human Dermal Fibroblasts) are fibroblasts derived from adult human skin, and the cells used for this Example were a commercially available product (Thermo Fisher Scientific, C0135C). hADSC cells (Human Adipose-derived stem cells) are mesenchymal stem cells derived from tissue obtained by human liposuction, and the cells used for this Example were a commercially available product (Thermo Fisher Scientific, R7788115).

### <Maintenance culture>

The cultured cells were washed twice with PBS, 0.5 mL of trypsin was added, and the cells were incubated for 5 minutes at 37°C, CO₂. After adding 3 mL of HDF medium (500 mL DMEM High Glucose, 55 mL FBS (10%), 2 ME; same for both HDF and hADSC), the cells were collected and centrifuged for 3 minutes at 1300 rpm, and again collected. The cells were passaged 1-3 times in HDF medium.

### <Induction of naive cells from somatic cells>

The surfaces of maintenance cultured cells were washed, and then washed twice with PBS, 0.5 mL of trypsin was added and the cells were incubated for 5 minutes at 37°C, CO₂. After adding 3 mL of HDF medium (500 mL DMEM High Glucose, 55 mL FBS (10%), 2 ME), the cells were collected and centrifuged for 3 minutes at 1300 rpm, and again collected. The cells were seeded in a 24-well plate at 5 x 10³ cells/well.

### <Transfection>

On the following day (Day 1), the cells were washed twice with PBS. The medium was exchanged with AK02 medium. mRNA and miRNA were prepared for transfection from Day 1 to 4. Table 5 shows the included factors. The miRNA Mimic shown in Table 5 are all a commercially available product (mirVana^{™} miRNA Mimic (Thermo Fisher)). A method of introducing a reprogramming factor into cells as an mRNA molecule is referred to as the "mRNA method". The reagents were combined as shown in Table 4. Although a commercial kit (such as StemRNA-3rd Gen Reprogramming Kit (Reprocell; 00-0076)) may be used instead of mixing the reagents, since such kits do not include miRNA Mimic hsa-miR-98-5p, the miRNA Mimic is preferably added separately. The cells were incubated at room temperature for 5 minutes. A and B were mixed by tapping and reacted at room temperature for 15 minutes. The reagents were added to each well and cultured overnight. On the following day, the aforementioned transfection was repeated up until Day 4. Maintenance culture was carried out with cRM2 + Y-27632 from Day 5. Naive-like cells appeared at around Days 7 to 9 (Fig. 23). Subsequent culturing was by the same procedure as from the 1^{st} passage of feeder-free culturing of the naive iPS cells of Example 1.

While the data is not shown here, even when the RNA cocktail shown in Table 5 with hsamiR-98-5p removed was used to induce naive pluripotent stem cells from somatic cells, naive-like cells appeared at Days 7 to 9. However, using hsa-miR-98-5p increased the induction effect for naive cells to a notable degree (about 3 to 10-fold).

### (Options)

On Day 5, the miRNA Mimic hsa-miR-98-5p shown in Table 5 may be added to cRM2 + Y-27632. The reagents were prepared as shown in Table 6 (preparation of 98-5p) and added dropwise to the culture solution. On the following day after overnight culturing, the medium was exchanged with cRM2 medium + Y-27632 (final concentration: 10 µM). As a result, it was possible to more effectively induce a naive-like dome-shaped cell mass (Fig. 24, left).

**[Table 4]**

| 24-well plate transfection | |
|---|---|
| (A) | Per 1 well/day |
| RNA cocktail | 3.35 µl (100 ng/µL) |
| OptiMEM | 46.65 µL |
| Total | 50 µL |
| | |

| (B) | Per 1 well (µL) |
|---|---|
| Lipofectamine RNAiMax (ThermoFisher Scientific 13778075) | 1.2 µL |
| OptiMEM | 48.8 µL |
| Total | 50 µL |

**[Table 5]**

| RNA cocktail | | | | | |
|---|---|---|---|---|---|
| OCT4 mRNA | SOX2 mRNA | KLF4 mRNA | MYCL mRNA | NANOG mRNA | LIN28A mRNA |
| E3L mRNA | K3L mRNA | B18 mRNA | | | |
| miRNA Mimic hsa-miR-302a-3p | miRNA Mimic hsa-miR-302b-3p | miRNA Mimic hsa-miR-302c-3p | miRNA Mimic hsa-miR-302d-3p | miRNA Mimic hsa-miR-367-3p | miRNA Mimic hsa-miR-98-5p (98-5p miRNA) |

**[Table 6]**

| Preparation of 98-5p miRNA | | |
|---|---|---|
| **Preparation of RNA solution** | µL | |
| 10 µM mimic nega/98-5p | 0.16 | |
| OptiMEM | 59.84 | |
| Total | 60 | |

| **Preparation of lipofectamine solution** | | µL |
|---|---|---|
| RNAiMAX | | 3 |
| OptiMEM | | 57 |
| Total | | 60 |

Naive pluripotent stem cells were directly induced from somatic cells, and then induced to primed pluripotent stem cells by the method described in Example 2 (Fig. 25, right, "primed pluripotent stem cells"). The primed pluripotent stem cells obtained by way of naive pluripotent stem cells differentiated to PGCLC (CD326+ CD49f+ cells) by the method described in Example 3 with high efficiency (Fig. 25) compared to the primed pluripotent stem cells induced from somatic cells without transitioning through naive pluripotent stem cells (Fig. 25, left).

This indicated that reprimed pluripotent stem cells and primed pluripotent stem cells derived from directly induced naive pluripotent stem cells both have low differentiation resistance.

### Example 5: Induction to naive pluripotent stem cells (reset cells) from somatic cells by way of primed pluripotent stem cells

In Example 1, naive pluripotent stem cells were induced from primed pluripotent stem cells. In this Example, primed pluripotent stem cells were induced from somatic cells, and then induced to naive pluripotent stem cells.

Human peripheral blood mononuclear cells (PBMC, Lonza, CC-2704) were used as the somatic cells. PBMCs were induced to primed pluripotent stem cells by the mRNA method using the RNA cocktail shown in Table 5, after culturing for 6 days in StemSpan^{™}-ACF Erythroid Expansion Medium (StemCell Technologies, ST-09860) containing preculture medium (100 ng/mL SCF (STEMCELL Technologies), 100 ng/mL TPO, 100 ng/mL Flt3, 50 ng/mL IL-6, 50 ng/mL IL-3, all by Fujifilm Co.). They were then induced to naive pluripotent stem cells (Day 0) according to the method of Example 1 (Induction of naive pluripotent stem cells in freeder-free conditions). Fig. 26 shows phase contrast microscope images up to 19 Days (Day 19). On Day 13, numerous typical naive pluripotent stem cell colonies were observed.

This demonstrated that primed pluripotent stem cells are induced from PBMCs by the mRNA method, and that naive pluripotent stem cells are subsequently produced satisfactorily by chemical resetting.

### Example 6: Variation of method for producing primed pluripotent stem cells derived from directly induced naive pluripotent stem cells

In Example 4, it was shown that inducing to primed pluripotent stem cells after first inducing to naive pluripotent stem cells can produce primed pluripotent stem cells with drastically reduced differentiation resistance compared to directly producing primed pluripotent stem cells from somatic cells, as primed pluripotent stem cells directly induced from somatic cells. This effect is not limited to the embodiment described in Example 4, as confirmed below.

### 1. Variation within let-7-5p miRNA family

It was confirmed whether naive pluripotent stem cells are still induced in the step of directly inducing naive pluripotent stem cells from somatic cells, when using other miRNA belonging to the let-7-5p miRNA family instead of miR-98-5p.

Three different HFD lines (one neonatal-derived, 2 adult-derived) were induced to naive pluripotent stem cells under the conditions of Example 4, except for changing the has-miR-98-5p in Table 5 to the miRNA mimic has-let-7a-5p or has-let-7b-5p (both mirVanaTM miRNA Mimic by Thermo Fisher). Adult HDF: Adult HDF1 (Promocell, C-12302) and Adult HDF2 (Thermo Fisher Scientific, R7788115), of which Adult HDF2 is the cell line used in Example 4. The same extent of typical naive pluripotent stem cell colonies was observed from all of the cell lines using let-7a-5p or let-7b-5p (Fig. 27, left image), similar to when miR-98-5p was used. When expression of representative pluripotent stem cell marker genes (OCT3/4, KLF4, NANOG) was analyzed by qPCR, all three marker genes were confirmed to be expressed at the same level in the cells induced using let-7a-5p and let-7b-5p, similar to the cells induced using miR-98-5p (Fig. 27, right graph).

It was thus demonstrated that not only miR-98-5p but also other miRNA belonging to the let-7-5p miRNA family have an effect of promoting induction from somatic cells to naive pluripotent stem cells.

### 2. Variation of somatic cells

It was confirmed that naive pluripotent stem cells can be induced by the method of Example 4 not only from HDF cells and hADSC cells but also from human umbilical vein endothelial cells (HUVEC).

HUVEC (C-12200 by Takara Bio, Inc.) were maintenance cultured on a cell culturing plate coated with collagen I, using endothelial cell growth medium (Endothelial Cell Growth Medium 2: EGM2 medium, C-22111 by Takara), and then transfected according to Example 4. EGM2 medium was used from Day 0 to Day 2, and AK03 medium was used from Day 3 to Day 5. A phase contrast microscope image at Day 14 is shown in the top left panel of Fig. 28. Numerous typical naive pluripotent stem cell colonies were observed.

The method of Example 2 was then carried out for induction to primed pluripotent stem cells. A phase contrast microscope image after 14 days from the start of induction to the primed type is shown at the top right panel of Fig. 28. Numerous colonies were observed, all of which were typical primed pluripotent stem cell colonies.

It was thus demonstrated that, even with HUVEC, primed pluripotent stem cells are satisfactorily produced from naive pluripotent stem cells obtained by the method of Example 4.

The effect of bone morphogenetic protein was analyzed.

At 6 days after introducing the RNA cocktail in Table 5 to HUVEC by the method of Example 4 (Day 6), 10 ng/mL of BMP4 (R&D, 314-BP-500, Recombinant human BMP4 Protein) was added to the medium, and culturing was carried out in BMP4-containing medium up to Day 7. As a result, on Day 14, more naive pluripotent stem cell colonies were obtained compared to the cell group without addition of BMP4 (data not shown).

Separately, a cocktail prepared by removing only has-miR-98-5p miRNA mimic from the RNA cocktail of Table 5 was used for an experiment of culturing with addition of 10 ng/mL BMP4 to the medium from Day 6, and culturing in BMP4 up to Day 7. The results are shown in the bottom row of Fig. 28. The number of naive pluripotent stem cell colonies was significantly reduced (bottom left) compared to the results using the RNA cocktail (containing miR-98-5p) in the top row, but it was confirmed that primed pluripotent stem cells were satisfactorily induced from the cells (bottom right).

It was thus demonstrated that bone morphogenetic protein also has an effect of promoting induction from somatic cells to naive pluripotent stem cells (although not as strong as miRNA belonging to the let-7-5p miRNA family), and that the promoting effect is even further increased by using bone morphogenetic protein in combination with miRNA belonging to the let-7-5p miRNA family.

### 3. Variation to method of inducing reprogramming factors

In Example 4, the naive pluripotent stem cells were induced from somatic cells by a method of introducing the reprogramming factor into the cells as an mRNA molecule (i.e. the mRNA method), rather than in a form encoded in an expression vector. It was therefore confirmed that it is possible to produce primed pluripotent stem cells with markedly improved differentiation resistance, not only by the mRNA method, but even when inducing naive pluripotent stem cells using an expression vector, and particularly using Sendai virus vector which is suitable from the viewpoint of regenerative medicine. It was also confirmed that primed pluripotent stem cells with even further improved differentiation resistance can be produced by sorting out only the CD320-high positive cells after inducing naive pluripotent stem cells, and inducing them to primed pluripotent stem cells.

Reprogramming using Sendai virus (SeV) vector was carried out with 2.0 L CytoTune (registered trademark)-iPS (ID Pharma). The Sendai virus vector in the kit encodes Oct3/4, Sox2, KLF4 and L-MYC as reprogramming factors (but does not encode miRNA belonging to the let-7-5p miRNA family).

After culturing PBMCs (Lonza, CC-2704) for 5 days in the preculture medium described in Example 5, they were seeded in a 24-well plate coated with iMatrix-511 silk at a cell density of 1 x 10⁵ cells/well. On the following day (Day 0), Sendai virus vector was contacted with the PBMCs according to the manual provided with the 2.0 L CytoTune (registered trademark)-iPS (ID Pharma). On Day 2, the medium was exchanged with AK03, and on Day 6 it was exchanged with cRM2 medium, carrying out culturing thereafter according to the method of Example 1 to induce naive pluripotent stem cells (Fig. 29, bottom left). A portion of the obtained naive pluripotent stem cells were induced to primed pluripotent stem cells by the method of Example 2 (Fig. 29, bottom right). The remaining naive pluripotent stem cells were provided for flow cytometry by the same method as "4. Verification of differentiation potency to primitive endoderm and miRNA expression" of Example 1 (Fig. 7D) to obtain a CD320-high positive cell fraction (Fig. 30, CD320⁺ cells). Cells before flow cytometry were used as the unsorted fraction (Fig. 30, Bulk).

Separately, the medium was exchanged with AK03 on Day 2 and culturing was carried out thereafter in AK03 medium for induction to primed pluripotent stem cells (Fig. 29, top).

The CD320-high positive cell fraction and the unsorted fraction were induced to primed pluripotent stem cells according to Example 2, after which they were induced to differentiate into primordial germ cells by the same method described under "2. Inducing human primordial germ cell-like cells (hPGCLC)" in Example 3 (Fig. 30). The differentiation efficiency to primordial germ-like cells was analyzed by flow cytometry (Fig. 31, left), and graphed (Fig. 31, right). With the primed pluripotent stem cells derived from unsorted naive pluripotent stem cells, the proportion of cells differentiated to primordial germ cell-like cells was about 8.68%, whereas the proportion was higher at 18% with the primed pluripotent stem cells derived from CD320-high positive naive pluripotent stem cells. While the results are not shown here, no cells were observed to differentiate into primordial germ-like cells with primed pluripotent stem cells directly induced from PBMCs (without transitioning to the naive state).

The residual amount of Sendai virus vector in the CD320-high positive cell fraction and the unsorted fraction at the start of inducing differentiation to primordial germ cells was analyzed by an established method (Fig. 29D). With iPSCs produced by the SeV method, differentiation is usually induced after adequate passages so that the Sendai virus vector introduced into the cells is sufficiently diluted. This is to maximally eliminate the effects of reprogramming factor that may be expressed from the residual Sendai virus vector.

As shown in Fig. 29D, a very large amount of Sendai virus vector was detected in the cells after 1 day from the start of reprogramming (Day 1) (Repro. Day 1), but virtually none was detected in the cells that had passed through 10 passages after induction to primed iPSCs (*primed iPSC (passage 10^{th})). In contrast, a considerable amount of Sendai virus vector was detected in both the CD320-high positive cell fraction and the unsorted fraction (Donor 1, 2 Bulk and CD320+).

It was thus shown that pluripotent stem cells obtained by inducing naive pluripotent stem cells from somatic cells and then inducing those cells to primed pluripotent stem cells (primed pluripotent stem cells derived from directly induced naive pluripotent stem cells) exhibit high differentiation potency even with a considerable amount of residual Sendai virus vector (reprogramming factor). The results further suggested that even higher differentiation potency is exhibited when the subculturing is carried out with the reprogramming factor in an adequately diluted state.

This, in combination with the results of "4. Verification of differentiation potency to primitive endoderm and miRNA expression" (Fig. 7) in Example 1, demonstrate that primed pluripotent stem cells with greatly improved differentiation resistance can be obtained by sorting out cells with high CD320 expression levels, regardless of whether the naive pluripotent stem cells were induced from somatic cells or primed pluripotent stem cells.

### INDUSTRIAL APPLICABILITY

According to the present invention it is possible to produce pluripotent stem cells with superior differentiation potency. It is possible to increase the differentiation potency even of pluripotent stem cells having low differentiation potency, thus reducing the difference in differentiation potency between existing lines of pluripotent stem cells, and especially iPS cells, and facilitating efforts to induce differentiation into a variety of cell types. Such differentiation-induced cells are useful for immunotherapy, regenerative medicine, innovative drug discovery screening, and so on.

The present application claims priority based on Japanese Patent Application No. 2023-032539 (filed in Japan on March 3, 2023), the entirety of the disclosure of which is incorporated herein by reference.

## Claims

1. A method for producing a primed pluripotent stem cell, comprising:
(1) a step of preparing a primate naive pluripotent stem cell, and
(2) a step of inducing a primed pluripotent stem cell from the naive pluripotent stem cell prepared in step (1).

2. The method according to claim 1, comprising a step of collecting the primed pluripotent stem cell obtained in step (2).

3. The method according to claim 1 or 2, wherein step (1) is a step of culturing a primate primed pluripotent stem cell to produce a naive pluripotent stem cell.

4. The method according to claim 3, wherein the culturing is carried out for 14 days or longer.

5. The method according to claim 3 or 4, comprising a step of sorting a CD320-positive cell from the cell population.

6. The method according to claim 1 or 2, wherein step (1) is a step of producing a naive pluripotent stem cell from a somatic cell without transitioning through a primed pluripotent stem cell.

7. The method according to claim 6, comprising a step of introducing a reprogramming factor and a miRNA belonging to the let-7-5p miRNA family into a somatic cell.

8. The method according to claim 7, wherein the miRNA belonging to the let-7-5p miRNA family is at least one type of miRNA selected from the group consisting of miR-98-5p, let-7a-5p, let-7b-5p, let-7c-5p, let-7e-5p, let-7f-5p, let-7g-5p and let-7i-5p.

9. The method according to any one of claims 6 to 8, comprising a step of introducing a bone morphogenetic protein into a cell.

10. The method according to any one of claims 6 to 9, comprising a step of activating a bone morphogenetic protein in a cell.

11. The method according to any one of claims 6 to 10, comprising a step of introducing a viral vector having a reprogramming factor into a somatic cell.

12. The method according to any one of claims 1 to 11, wherein step (2) comprises a step of culturing the naive pluripotent stem cell prepared in step (1) for 4 days or longer in medium containing bFGF.

13. The method according to any one of claims 1 to 12, wherein step (2) is culturing under a feeder-free condition.

14. The method according to any one of claims 1 to 13, wherein the entire culturing period is under feeder-free and xeno-free conditions.

15. The method according to any one of claims 1 to 14, wherein the pluripotent stem cell is an induced pluripotent stem cell.

16. A primed pluripotent stem cell obtained by the method according to any one of claims 1 to 15.

17. A method for producing a cell or an organoid, comprising a step of inducing differentiation of the primed pluripotent stem cell according to claim 16.

18. The method according to claim 17, wherein the cell is selected from the group consisting of a neural crest cell, a neural progenitor cell, a neuron, a hematopoietic progenitor cell, a platelet, a T cell, an epiblast-like cell, a primordial germ cell and a myocardial cell.

19. The method according to claim 17, wherein the organoid is a cerebral cortex organoid.

20. A method for increasing the differentiation potency of a pluripotent stem cell, comprising:
(1) a step of inducing a naive pluripotent stem cell from a primate primed pluripotent stem cell, and
(2) a step of inducing a primed pluripotent stem cell from the naive pluripotent stem cell induced in step (1).
